# EUROPEAN PATENT SPECIFICATION

(11) **EP 1 187 653 B1**
(45) Date of publication and mention of the grant of the patent: **31.03.2010**
(21) Application number: 00939538.5
(22) Date of filing: 02.06.2000
(51) Int. Cl.: A61M 37/00, A61B 10/00

(54) **DEVICES FOR ENHANCED MICRONEEDLE PENETRATION OF BIOLOGICAL BARRIERS**
VORRICHTUNGEN ZUR VERGRÖSSERTEN PENETRATION VON MIKRONADELN IN BIOLOGISCHEN HAUTSCHICHTEN
DISPOSITIFS PERMETTANT D'AMELIORER LA PENETRATION D'UNE MICROAIGUILLE A TRAVERS DES BARRIERES TISSULAIRES

(30) Priority: 04.06.1999 US 137621 P; 29.07.1999 US 146200 P; 23.11.1999 US 448107; 02.12.1999 US 452979; 02.12.1999 US 453109
(43) Date of publication of application: 20.03.2002
(73) Proprietor: GEORGIA TECH RESEARCH CORPORATION, Atlanta, GA 30332-0415 (US)
(72) Inventor: PRAUSNITZ, Mark, R., Decatur, GA 30030 (US); ALLEN, Mark, G., Atlanta, GA 30328 (US); HENRY, Sebastien, Smyrna, GA 30080 (US); MCALLISTER, Devin, V., Holley, NY 14470 (US); ACKLEY, Donald, E., Cardiff, CA 92007 (US); GUJRAL, Inder-Jeet, Cambridge, MA 02138 (US); JACKSON, Thomas, La Jolla, CA 92037 (US)
(74) Representative: Stevens, Ian Edward
(86) International application number: PCT/US2000/015312
(87) International publication number: WO 2000/074763

(56) References cited:
- EP-A- 0 497 620
- WO-A-98/00193
- WO-A-98/00194
- WO-A-98/28037
- WO-A-99/64580
- DE-A- 19 525 607
- US-A- 3 964 482
- US-A- 5 865 786

## Description

### Background Of The Invention

This invention is generally in the field of devices for the transport of therapeutic or biological molecules across tissue barriers, such as for drug delivery or for the withdrawal and sensing of biological fluids, such as in the analysis of blood glucose levels.

A frequent limitation of drugs and therapeutic agents is their delivery: how to transport drugs across biological barriers in the body (e.g.. the skin, the oral mucosa, the blood-brain barrier), which normally do not transport drugs at rates that are therapeutically useful or optimal.

Drugs commonly are administered orally as pills or capsules. Many drugs, however, cannot be effectively delivered in this manner, due to degradation in the gastrointestinal tract and/or elimination by the liver. Moreover, some drugs cannot effectively diffuse across the intestinal mucosa. Patient compliance may also be a problem in therapies requiring, for example, that pills be taken at particular intervals over a prolonged time.

Another common technique for delivering drugs across a biological barrier is the use of a needle, such as those used with standard syringes or catheters, to transport drugs across (through) the skin. While effective for this purpose, needles generally cause pain; local damage to the skin at the site of insertion; bleeding, which increases the risk of disease transmission: and a wound sufficiently large to be a site of infection. Needle techniques also generally require administration by one trained in its use, and are not preferred for frequent routine use due to the vascular damage caused by repeated punctures, nor are they desirable for long term, controlled continuous drug delivery.

Similarly, current methods of sampling biological fluids are invasive and suffer from the same disadvantages. For example, the extraction of analytes through the skin is critical to diabetic patients, who typically must measure blood glucose several times per day in order to optimize insulin treatment and thereby reduce the severe long-term complications of the disease. Currently, diabetics do this by pricking the highly vascularized fingertips with a lancet to perforate the skin, then milking the skin with manual pressure to produce a drop of blood, which is then assayed for glucose using a disposable diagnostic strip and a meter into which the strip fits. This method of glucose measurement has the major disadvantage that it is painful, so diabetics do not like to obtain a glucose measurement as often as is medically indicated. It would therefore be highly useful to be able to obtain a sample of blood, lymph, or interstitial fluid more quickly, using an easier procedure, and relatively noninvasively. It also would be advantageous to be able to repeatedly or continually extract analyte transdermally over a period of time

An alternative delivery technique is the transdermal patch, which usually relies on diffusion of the drug across the skin. However, this method is not useful for many drugs, due to the poor permeability (i.e. effective barrier properties) of the skin. The rate of diffusion depends in part on the size and hydrophilicity of the drug molecules and the concentration gradient across the stratum corneum. Few drugs have the necessary physiochemical properties to be effectively delivered through the skin by passive diffusion. Iontophoresis, electroporation, ultrasound, and heat (so-called active systems) have been used in an attempt to improve the rate of delivery. While providing varying degrees of enhancement, these techniques are not suitable for all types of drugs, failing to provide the desired level of delivery. In some cases, they are also painful and inconvenient or impractical for continuous controlled drug delivery over a period of hours or days. Some proposed alternatives to the needle require the use of (1) lasers or heat to create a hole in the skin, which is inconvenient, expensive, or undesirable for repeated use; (2) electric fields or ultrasound, which also is inconvenient and expensive; or (3) chemical or biological penetration enhancing agents, which can be irritating to the tissue and undesirable for repeated use. Attempts have been made to design alternative devices for active transfer of drugs, or analyte to be measured, through the skin.

For example, U.S. Patent No. 5,879,326 to Godshall et al. and PCT WO 96/37256 by Silicon Microdevices, Inc. disclose a transdermal drug delivery apparatus that includes a cutter portion having a plurality of microprotrusions, which have straight sidewalls, extending from a substrate that is in communication with a drug reservoir. In operation, the microprotrusions penetrate the skin until limited by a stop region of the substrate and then are moved parallel to the skin to create incisions. Channels in the substrate adjacent to the microprotrusions allow drug from the reservoir to flow to the skin near the area disrupted by the microprotrusions. Merely creating a wound, rather than using a needle which conveys drug through an enclosed channel into the site of administration, creates variability in dosage.

U.S. Patent No. 5,250.023 to Lee et al*.* discloses a transdermal drug delivery device which includes a plurality of needles having a diameter in the range of 50 to 400 µm. The needles are supported in a water-swellable polymer substrate through which a drug solution permeates to contact the surface of the skin. An electric current is applied to the device to open the pathways created by the needles, following their withdrawal from the skin upon swelling of the polymer substrate.

PCT WO 93/17754 by Gross et al*.* discloses another transdermal drug delivery device that includes a housing having a liquid drug reservoir and a plurality of tubular elements for transporting liquid drug into the skin. The tubular elements may be in the form of hollow needles having *inner* diameters of less than I mm and an outer diameter of 1.0 mm.

While each of these devices has potential use, there remains a need for better drug delivery devices, which make smaller incisions, deliver drug with greater efficiency (greater drug delivery per quantity applied) and less variability of drug administration, and/or are easier to use. In view of these needs, microneedle devices have been developed, which are described in PCT WO 99/64580. Certain embodiments of the device were found to readily penetrate skin samples in *in vitro* experiments, but not always provide uniform or complete insertion of the microneedles into some areas of the skin *in vivo*, as the stratum corneum and underlying tissues are highly deformable and elastic over much of the body.

US 6,132,755 discloses a transcorneal drug-release system for delivering a medicament which comprises a plurality of micro-pins or micro-blades.

WO 98/00194 describes methods and devices for application of ultrasound for enhancing transdermal transport.

It is therefore an object of the present invention to provide a microneedle device for relatively painless, controlled, safe, convenient transdermal delivery of drugs.

It is a further object of the present invention to provide a microneedle device controlled sampling or sensing of biological fluids in a minimally-invasive, painless, and convenient manner.

It is another object of the present invention to provide methods and devices for improving the control of microneedle insertion into the body of a patient.

### Summary Of The Invention

According to the invention, there is provided a device as defined in the appended Claim 1. Preferred embodiments of the device are defined in Claims 2 to 36.

Microneedle devices are provided for delivery of drugs across or into biological tissue and for controlled sampling of biological fluids in a minimally-invasive, painless, and convenient manner. The microneedle devices permit drug delivery at clinically relevant rates across or into skin or other tissue barriers.

In a preferred embodiment for drug delivery, the devices include a plurality of hollow microneedles, which are attached to or integrated into a substrate, and at least one reservoir selectably in communication with the microneedles, wherein the volume or amount of drug to be delivered can be selectively altered. The reservoir contains the drug to be delivered, and can be formed of a deformable, preferably elastic, material. The device also can include means for compressing the reservoir to drive the drug from the reservoir through the microneedles. The means can include a plunger or osmotic pump. In one embodiment, the reservoir is a syringe or pump connected to the substrate. The device also can include a sealing mechanism to contain the drug in one or more of the reservoirs until it is ready to be delivered or mixed with a liquid carrier. In one embodiment, the sealing mechanism is a fracturable barrier interposed between the reservoir and the substrate. The device also can include a means for providing feedback, such as color change, to the user to indicate that delivery has been initiated and/or completed. In another embodiment, the microneedle device further includes a rate control means, such as a semi-permeable membrane, to regulate the rate or extent of drug which flows through the microneedles.

The microneedle devices preferably are provided with means for preventing undesired reuse of or contact with the microneedles. These means can include protective packaging, such as a peelable liner that temporarily covers the tips of the microneedles. The packaging also can be used to shear off the microneedles following their intended use, thereby preventing their reuse.

Also provided are microneedle devices and methods of use thereof for the enhanced transport of molecules, including drugs and biological molecules, across tissue by improving the interaction of an array of microneedles and a deformable, elastic biological barrier, such as human skin. The devices and methods act to (1) limit the elasticity. (2) adapt to the elasticity. (3) utilize alternate ways of creating the holes for the microneedles to penetrate the biological barrier, other than the simply direct pressure of the microneedle substrate to the barrier surface, or (4) combinations of (1)-(3).

In preferred embodiments for limiting the elasticity of skin, the microneedle device includes features suitable for stretching, pulling, or pinching the skin to present a more rigid, less deformable, surface in the area to which the microneedles are applied (i.e. penetrate). For example, a vacuum can be applied to the area of the skin at the site of microneedle application to pull it taut and/or pull the skin onto the tips of the microneedles. Alternatively or in addition, the elasticity of skin can be reduced by applying a thin film or membrane over the skin surface at the site of application, so as to keep the skin tight, limiting the ability of the skin to stretch at the application site. The microneedles are then pushed through the film or membrane and into the skin.

In preferred embodiments for adapting the device to the elasticity of skin, the microneedles of the device include individual extensions or are provided in a curved three dimensional array, for example, by using a flexible substrate and/or varying the height of the microneedles in the array. In another embodiment, the microneedles are applied to the skin surface at an increased velocity, thereby reducing the time available for the stratum corneum and underlying tissues to deform from contact with the tips or entire length of the microneedles.

In a preferred embodiment for creating holes in the skin for microneedles, tiny holes are burned into the skin, for example, by heating of the tips of the microneedles and/or by using a laser. In another embodiment, a focused blast of high pressure gas is used to create the holes.

### Brief Description Of The Drawings

Figures 1a-e are side cross sectional views of a method for making hollow microneedles.
Figures 2a-g are side cross-sectional views of a method for making a hollow microneedle.
Figures 3a-d are side cross-sectional views illustrating a preferred method for making hollow microneedles.
Figures 4a-d are side cross-sectional views illustrating a preferred method for making hollow silicon microtubes.
Figures 5a-e are side cross-sectional views illustrating a preferred method for making hollow metal microtubes.
Figures 6a-d are side cross-sectional views illustrating a preferred method for making tapered metal microneedles.
Figures 7a-d are side cross-sectional views illustrating a method for making tapered microneedles using laser-formed molds.
Figures 8a-f are side cross-sectional views illustrating a second method for making tapered microneedles using laser-formed molds.
Figure 9 is a side elevational view of a schematic of an embodiment of the microneedle device inserted into undeformed skin.
Figures 10a-d are illustrations of microneedle devices having various embodiments of microneedle extensions.
Figures 11a-c are cross-sectional views illustrating microneedle devices having curved substrates (11a), varying microneedle height (11b), and linear microneedle arrays (11c).
Figures 12a-c are cross-sectional views illustrating preferred microneedle devices inserted into skin with altered elasticity.
Figures 13a-c are cross-sectional views illustrating preferred embodiments of microneedle devices which can apply suction to skin at the site of insertion.
Figures 14a-c are cross-sectional (14a-b) and perspective (14c) views illustrating preferred embodiments of microneedle devices which stretch the skin at the site of insertion.
Figures 15a-c are cross-sectional views illustrating examples of microneedle devices which pinch the skin at the site of insertion.
Figure 16 is a cross-sectional view of a preferred embodiment of a microneedle device for use in localized heating of skin.
Figure 17 is a cross-sectional view of a preferred embodiment of a microneedle device having a microneedle reinforcing layer.
Figures 18a-b are cross-sectional views of a preferred embodiment of a microneedle device having a flexible substrate in an unactivated position (18a) and an activated position (18b).
Figure 19 is a cross-sectional view a preferred embodiment of a microneedle device having arrays of microneedles with spaces between the arrays.
Figures 20a-c are cross-sectional views of preferred embodiments of a microneedle drug delivery device. The device of Figure 20a includes a reservoir and is suitable for transdermal drug delivery. The device of Figures 20a and 20b includes a deformable reservoir, wherein delivery is activated by manual. e.g., finger or thumb, pressure applied to compress the reservoir directly (20b) or indirectly (20c).
Figure 21 is a cross-sectional view of another preferred embodiment of a microneedle drug delivery device, wherein delivery is activated by manual pressure applied via a plunger to compress the reservoir.
Figure 22 is a cross-sectional view of another preferred embodiment of a microneedle drug delivery device, wherein delivery is activated by releasing a compressed spring which forces the plunger to compress the reservoir.
Figures 23a-b are cross-sectional views of preferred embodiments of a microneedle drug delivery device having a multiple chambered reservoirs.
Figure 24 is a cross-sectional view of a preferred embodiment of the microneedle drug delivery device, which incorporates an osmotic pump to force the drug contents from the reservoir.
Figure 25 is a cross-sectional view of a preferred embodiment of a microneedle device for fluid withdrawal.
Figure 26 is a cross-sectional view of another preferred embodiment of a microneedle device for fluid withdrawal.
Figures 27a-c are cross-sectional views of preferred embodiments of a microneedle device in which a sensor is included on the external surface of a hollow needle (27a), on the internal bore surface of a hollow needle (27b), and within the pores of a porous, hollow needle (27c).

### Detailed Description Of The Invention

### 1. Biological Barriers

The devices disclosed herein are useful in transport of material into or across biological barriers including the skin (or parts thereof): the blood-brain barrier: mucosal tissue (e.g., oral, nasal, ocular, vaginal, urethral, gastrointestinal, respiratory); blood vessels: lymphatic vessels; or cell membranes (e.g., for the introduction of material into the interior of a cell or cells). The biological barriers can be in humans or other types of animals, as well as in plants, insects, or other organisms, including bacteria, yeast, fungi, and embryos. The microneedle devices can be applied to tissue internally with the aid of a catheter or laparoscope. For certain applications, such as for drug delivery to an internal tissue, the devices can be surgically implanted.

In a preferred embodiment, the microneedle device disclosed herein is applied to skin. The stratum corneum is the outer layer, generally between 10 and 50 cells, or between 10 and 20 µm thick. Unlike other tissue in the body, the stratum corneum contains "cells" (called keratinocytes) filled with bundles of cross-linked keratin and keratohyalin surrounded by an extracellular matrix of lipids. It is this structure that is believed to give skin its barrier properties, which prevents therapeutic transdermal administration of many drugs. Below the stratum corneum is the viable epidermis, which is between 50 and 100 µm thick. The viable epidermis contains no blood vessels, and it exchanges metabolites by diffusion to and from the dermis. Beneath the viable epidermis is the dermis, which is between 1 and 3 mm thick and contains blood vessels, lymphatics, and nerves.

As used herein, references to using the microneedle devices on "skin" also include using the microneedle devices with other biological barriers unless expressly limited to only skin.

### 2. The Microneedle Device

The microneedle devices disclosed herein include a substrate, one or more microneedles, and optionally (i) a reservoir for delivery of drugs, (ii) a fluid collection chamber for collection of biological fluid and/or (iii) a sensor for sensing an analyte, all or any of which (i-iii) can be selectably in communication with at least one microneedle. The devices also can include one or more pumps, sensors, and/or microprocessors to control the interaction of the foregoing. The microneedle device preferably includes penetration enhancing features to alter or adapt to deformable and elastic biological barrier, such as the skin over much of the human body. Preferably, the microneedles are provided as a multi-dimensional array, in contrast to a device with a single microneedle or single row of microneedles. The microneedle device can be adapted to be a single-use, disposable device, or can be adapted to be fully or partially reusable.

In one embodiment for delivery of drugs, the microneedle device includes at least three components; a plurality of microneedles; a substrate to which the base of the microneedles are secured or integrated: and at least one reservoir that is selectably in fluid connection with one or more of the microneedles.

### a. Substrate

The substrate of the device can be constructed from a variety of materials, including metals, ceramics, semiconductors, organics, polymers, and composites. The substrate includes the base to which the microneedles are attached or integrally formed. A reservoir and/or fluid collection chamber also may be attached to, or integrally formed with, the substrate. The substrate can be adapted to fit a Luer-Lock syringe or other conventionally used drug delivery or fluid withdrawal device that currently uses hypodermic needles as the barrier penetration method. A fluid collection chamber and/or sensor can be attached to the substrate or formed (e.g., as part of the substrate) to communicate directly with the base of the microneedles.

In one embodiment of the device, the substrate is formed from a thin, rigid material that is sufficiently stiff so as to force the attached microneedles through the biological barrier in such areas where the barrier resists deformation by the microneedles.

In a preferred embodiment of the device, the substrate, and possibly other components, is formed from flexible materials, or have appropriate materials so as to be flexible, to allow the device to fit the contours of the biological barrier (such as the skin, vessel walls, or the eye) to which the device is applied and to adapt to barrier deformation that may occur when the microneedle device is applied. A flexible device further facillitates more consistent penetration during use, since penetration can be limited by deviations in the attachment surface. For example, the surface of human skin is not flat due to dermatoglyphics, i.e. tiny wrinkles, and hair, and is highly deformable. The flexible substrate can be deformed mechanically (for example, using an actuator or simply by fluid pressure) in order to pierce the biological barrier. Conformality of the flexible device to the skin can be further enhanced by using an external force, such as a uniform pressure, to aid insertion of the microneedles into the biological barrier. However, for some biological barriers or applications, a rigid substrate may be preferred.

### b. Microneedle

The microneedles of the device can be constructed from a variety of materials, including metals, ceramics, semiconductors, organics, polymers, and composites. Representative materials of construction include pharmaceutical grade stainless steel, gold, titanium, nickel, iron, gold, tin, chromium, copper, palladium, platinum, alloys of these or other metals, silicon, silicon dioxide, and polymers. Representative biodegradable polymers include polymers of hydroxy acids such as lactic acid and glycolic acid polylactide, polyglycolide, polylactide-co-glycolide, and copolymers with PEG, polyanhydrides, poly(ortho)esters, polyurethanes, poly(butyric acid), poly(valeric acid), and poly(lactide-co-caprolactone). Representative non-biodegradable polymers include polycarbonate, polymethacrylic acid, ethylenevinyl acetate, polytetrafluoroethylene (TEFLON^{™}), and polyesters.

- Generally, the microneedles should have the mechanical strength to remain intact for delivery of drugs, or serve as a conduit for the collection of biological fluid, while being inserted into the skin, while remaining in place for up to a number of days, and while being removed. In embodiments where the microneedles are formed of biodegradable polymers, however, this mechanical requirement is less stringent, since the microneedles or tips thereof can break off, for example in the skin, and will biodegrade. Nonetheless, even a biodegradable microneedle still needs to remain intact at least long enough for the microneedle to serve its intended purpose (e.g., its conduit function). Therefore, biodegradable microneedles can provide an increased level of safety, as compared to nonbiodegradable ones. The microneedles generally should be sterilizable using standard methods such as ethylene oxide or gamma irradiation.

The microneedles can be formed of a nonporous solid, a porous solid (with or without a sealed coating or exterior portion), or hollow. As used herein, the term "porous" means having pores or voids throughout at least a portion of the microneedle structure, sufficiently large and sufficiently interconnected to permit passage of fluid and/or solid materials through the microneedle. As used herein, the term "hollow" means having one or more substantially annular bores or channels through the interior of the microneedle structure, having a diameter sufficiently large to permit passage of fluid and/or solid materials through the microneedle. The annular bores may extend throughout all or a portion of the needle in the direction of the tip to the base, extending parallel to the direction of the needle or branching or exiting at a side of the needle, as appropriate. A solid or porous microneedle can be hollow. One of skill in the art can select the appropriate porosity and/or bore features required for specific applications. For example, one can adjust the pore size or bore diameter to permit passage of the particular material to be transported through the microneedle device. The inner surface of the bore of hollow microneedles can be made rough to enhance cell membrane disruption for those applications in which cell disruption is useful.

The microneedles can have straight or tapered shafts. A hollow microneedle that has a substantially uniform diameter, which needle does not taper to a point, is referred to herein as a "microtube." As used herein, the term "microneedle" includes both microtubes and tapered needles unless otherwise indicated. In one embodiment, the diameter of the microneedle is greatest at the base end of the microneedle and tapers to a point at the end distal the base. The microneedle can also be fabricated to have a shaft that includes both a straight (untapered) portion and a tapered portion.

In a preferred embodiment for drug delivery, the microneedles are hollow: that is, each contains at least one substantially annular bore or channel having a diameter large enough to permit passage of a drug-containing fluid and/or solid materials through the microneedle. The bores may be linear, i.e. extend upwardly from needle base to needle tip, or they may take a more complex path. e.g. extend upwardly from the needle base, but then lead to one or more 'portholes' or `slits' on the sides of the needles, rather than an opening at the needle tip.

The microneedles can be formed with shafts that have a circular cross-section in the perpendicular, or the cross-section can be non-circular. For example, the cross-section of the microneedle can be polygonal (e.g. star-shaped, square, triangular), oblong, or another shape. The shaft can have one or more bores. The cross-sectional dimensions typically are between about 10 nm and 1 mm, preferably between 1 and 500 µm, and more preferably between 10 and 200 µm. The outer diameter is typically between about 10 µm and about 100 µm, and the inner diameter is typically between about 3 µm and about 80 µm.

The length of the microneedles typically is between about 10 µm and 1 mm, preferably between 100 and 1 mm, more preferably between 100 and 500 µm, and more preferably between 150 and 350 µm. An array of microneedles can include a mixture of microneedles having, for example, various lengths, outer diameters, inner diameters, cross-sectional shapes, and spacing between the microneedles. Preferably, the microneedles are sized to avoid or minimize contact with nerve endings in the biological tissue, such as the dermis, thereby eliminating or reducing pain when the microneedles are inserted, for example into the skin.

The microneedles can be oriented perpendicular or at an angle to the substrate. Preferably, the microneedles are oriented perpendicular to the substrate to provide structural strength and to permit ease of insertion into the tissue. An array of microneedles can include a mixture of microneedle orientations, heights, spacings, or other parameters. This variation in an array can be useful, for example, if different microneedles are to provide different sensing or insertion functions.

In embodiments for sensing and/or withdrawal, the microneedles function as a conduit, a sensing element, or a combination thereof. In one embodiment, one or more of the microneedles are coated (if solid, porous, or hollow) and/or at least partially filled (if porous or hollow) with a sensing or diffusion-modifying material. In one embodiment, the microneedle can have one or more holes (ports) or slits positioned on the sides of the shaft of a hollow microneedle having a blunt, open, or closed tip. This design may reduce blockage of the conduit, for example, due to tissue or cells when the microneedles are inserted.

### c. Reservoir

In some embodiments, particularly those for drug delivery and fluid extraction, the microneedle device includes a reservoir in communication, preferably selectably so, with the microneedles. The reservoir can be attached to the substrate by any suitable means. In a preferred embodiment, the reservoir is attached to the back of the substrate (opposite the microneedles) around the periphery, using an adhesive agent (e.g., glue). A gasket may also be used to facilitate formation of a fluid-tight seal.

In a preferred embodiment, the reservoir contains drug, for delivery through the microneedles. The reservoir may be a hollow vessel, a porous matrix, or a solid form including drug which is transported therefrom. The reservoir can be formed from a variety of materials that are compatible with the drug or biological fluid contained therein. Preferred materials include natural and synthetic polymers, metals, ceramics, semiconductors, organics, and composites. In one embodiment, the reservoir is a standard syringe.

The microneedle device can include one or a plurality of chambers for storing materials to be delivered. In the embodiment having multiple chambers, each can be in fluid connection with all or a portion of the microneedles of the device array. In one embodiment, at least two chambers are used to separately contain drug (e.g.. a lyophilized drug, such as a vaccine) and an administration vehicle (e.g., saline) in order to prevent or minimize degradation during storage. Immediately before use, the contents of the chambers are mixed. Mixing can be triggered by any means, including, for example, mechanical disruption (i.e. puncturing or breaking), changing the porosity, or electrochemical degradation of the walls or membranes separating the chambers. In another embodiment, a single device is used to deliver different drugs, which are stored separately in different chambers. In this embodiment, the rate of delivery of each drug can be independently controlled.

In a preferred embodiment, the reservoir should be in direct contact with the microneedles and have holes through which drug could exit the reservoir and flow into the interior of hollow or porous microneedles. In another preferred embodiment, the reservoir has holes which permit the drug to transport out of the reservoir and onto the skin surface. From there, drug is transported into the skin, either through hollow or porous microneedles, along the sides of solid microneedles, or through pathways created by microneedles m the skin.

In a preferred embodiment for drug delivery, the reservoir is selectably in connection with the microneedle bore, such that the reservoir contents can flow from the reservoir and out through the microneedle tip, into the target tissue. Typically, it is attached to, or integrated into, the substrate, either integrally (as in a one-piece device) or at the moment of drug delivery (as with a Luer-lock type device). The reservoir is to provide suitable, leak-free storage of the drug composition before it is to be delivered. The reservoir should keep the drug composition free of contaminants and degradation-enhancing agents. For example, the reservoir should exclude light when the drug composition contains photo-sensitive materials, and should include an oxygen barrier material in order to minimize exposure of drugs sensitive to oxidation. Also, the reservoir should keep volatile materials inside the reservoir, for example, to prevent water from evaporating, causing the drug composition to dry out and become undeliverable.

The drug reservoir can be substantially rigid or readily deformable. The reservoir can be formed from one or more polymers, metals, ceramics, or combinations thereof. In a preferred embodiment, the reservoir includes a volume surrounded by one or more walls, or includes a porous material, such as a sponge, which can retain, for example, the drug liquid until the material is compressed.

In a preferred embodiment, the reservoir is formed of an elastic material, such as an elastomeric polymer or rubber. For example, the reservoir can be a balloon-like pouch that is stretched (in tension) when filled with a fluid drug composition to be delivered.

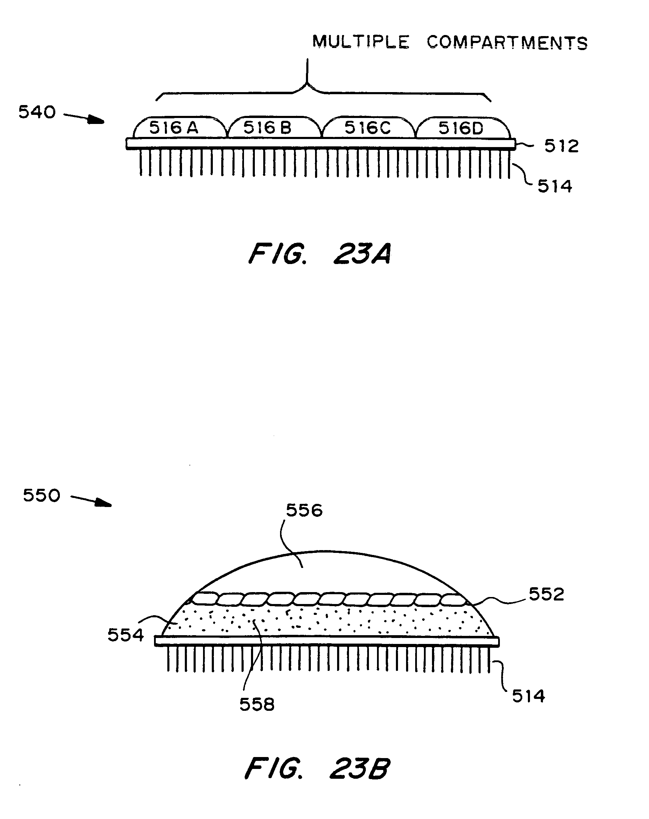

The reservoir of a single microneedle device can include a plurality of compartments that are isolated from one another and/or from a portion of the microneedles in an array. The device can, for example, be provided to deliver different drugs through different needles, or to deliver the same or different drugs at different rates or at different times (Figure 23a). Alternatively, the contents of the different compartments can be combined with one another, for example, by piercing, or otherwise removing, a barrier between the compartments, so as to allow the materials to mix. In a preferred embodiment, one compartment contains a saline solution or another delivery vehicle, while another compartment contains lyophilized drug (Figure 23b). In a preferred embodiment, the reservoir is a standard or Luer Lock syringe adapted to a microneedle array.

### d. Collection Chamber

In embodiments for extraction, the device preferably includes a fluid collection chamber. The fluid collection chamber is selectably in connection with the microneedle bores or pores, such that a biological fluid can flow from the tissue surrounding the microneedle, through the microneedle, and into the collection chamber. Typically, the collection chamber is attached to, or integrated into, the substrate. The chamber should function to contain a biological fluid sample so as to permit analysis within the microneedle device or following transfer to a separate analytical device.

The collection chamber can be substantially rigid or readily deformable. The collection chamber can be formed from one or more polymers, metals, ceramics, semiconductor, or combinations thereof. In a preferred embodiment, the collection chamber contains a porous or absorbent material, such as a sponge, gel, or paper or polymeric strip. The material can be permanently contained or removable, and can function as a diagnostic element or substrate for use in analytical devices. The chamber can initially be empty or can contain a gas or one or more reagents in any form (e.g.. liquid or solid particles). In one embodiment, at least a portion of the interior walls of the chamber are coated with a reagent for assaying the biological fluid.

In a preferred embodiment, the collection chamber is formed of an elastic material, such as an elastomeric polymer or rubber. For example, the collection chamber can be a collapsed balloon-like pouch that expands when the biological fluid is drawn into the collection chamber.

The collection chamber of a microneedle device can include a plurality of compartments that are temporarily or permanently isolated from one another and/or from a portion of the microneedles in an array. The device can, for example, be provided to collect or sense through different needles at different rates or at different times into the different compartments. Alternatively, some of the different compartments can contain analytical reagents which can be combined with the biological fluid sample, for example, by piercing, or otherwise removing, a barrier between the compartments, so as to allow the materials to mix for analysis.

In another preferred embodiment, the collection chamber is adapted to receive and use standard glucose sensing strips, wich can be loaded into the microneedle device before, during, or after the biological fluid is extracted.

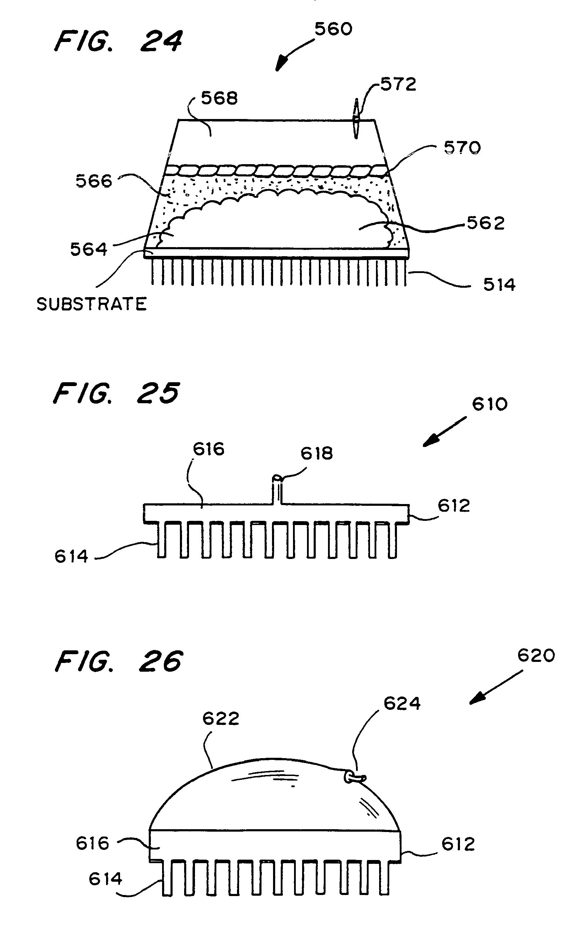

Two embodiments of the microneedle device are shown in Figures 25-26. In Figure 25. microneedle device **610** includes substrate **612** from which a three-dimensional array of microneedles **614** protrude. As shown, the annular bore of the microneedles **614** extends through the substrate **612.** The device **610** also includes fluid collection chamber **616** which is in fluid connection with the microneedles **614.** Fluid collection chamber **616** includes an access port **618** through which collected fluid can be withdrawn from collection chamber **616.** and/or through which a withdrawing force, such as a vacuum, can be applied. In Figure 26, microneedle device **620** includes substrate **612** from which a three-dimensional array of microneedles **614** protrude, and fluid collection chamber **616,** as well as elastic cap **622** and one-way valve **624.** The interior of elastic cap **622** is in communication with, or open to, fluid collection chamber **616.**

### e. Transport Control Components

The microneedle device also must be capable of transporting material across the barrier at a useful rate. For example, the microneedle device must be capable of delivering drug across the skin at a rate sufficient to be therapeutically useful. The device may include a housing with microelectronics and other micromachined structures to control the rate of delivery either according to a preprogrammed schedule or through active interface with the patient, a healthcare professional, or a biosensor. The rate can be controlled by manipulating a variety of factors, including the characteristics of the drug formulation to be delivered (e.g., its viscosity, electric charge, and chemical composition); the dimensions of each microneedle (e.g., its outer diameter and the area of porous or hollow openings); the number of microneedles in the device; the application of a driving force (e.g., a concentration gradient, a voltage gradient, a pressure gradient); and the use of a valve.

The rate also can be controlled by interposing between the drug in the reservoir and the opening(s) at the base end of the microneedle polymeric or other materials selected for their diffusion characteristics. For example, the material composition and layer thickness can be manipulated using methods known in the art to vary the rate of diffusion of the drug of interest through the material, thereby controlling the rate at which the drug flows from the reservoir through the microneedle and into the tissue.

Transportation of molecules through the microneedles can be controlled or monitored using, for example, various combinations of valves, pumps, sensors, actuators, and microprocessors. These components can be produced using standard manufacturing or microfabrication techniques. Actuators that may be useful with the microneedle devices disclosed herein include micropumps, microvalves, and positioners. In a preferred embodiment, a microprocessor is programmed to control a pump or valve, thereby controlling the rate of delivery.

Flow of molecules through the microneedles can occur based on diffusion, capillary action, or can be induced using conventional mechanical pumps or nonmechanical driving forces, such as electroosmosis or electrophoresis, or convection. For example, in electroosmosis, electrodes are positioned on the biological barrier surface, one or more microneedles, and/or the substrate adjacent the needles, to create a convective flow which carries oppositely charged ionic species and/or neutral molecules toward or into the biological barrier. In a preferred embodiment, the microneedle device is used in combination with another mechanism that enhances the permeability of the biological barrier, for example by increasing cell uptake or membrane disruption, using electric fields, ultrasound, chemical enhancers, vacuum viruses, pH, heat and/or light.

Passage of the microneedles, or drug to be transported via the microneedles, can be manipulated by shaping the microneedle surface, or by selection of the material forming the microneedle surface (which could be a coating rather than the microneedle per se). For example, one or more grooves on the outside surface of the microneedles can be used to direct the passage of drug, particularly in a liquid state. Alternatively, the physical surface properties of the microneedle can be manipulated to either promote or inhibit transport of material along the microneedle surface, such as by controlling hydrophilicity or hydrophobicity.

The flow of molecules can be regulated using a wide range of valves or gates. These valves can be the type that are selectively and repeatedly opened and closed, or they can be single-use types. For example, in a disposable, single-use drug delivery device, a fracturable barrier or one-way gate may be installed in the device between the reservoir and the opening of the microneedles. When ready to use, the barrier can be broken or gate opened to permit flow through the microneedles. Other valves or gates used in the microneedle devices can be activated thermally, electrochemically, mechanically, or magnetically to selectively initiate, modulate, or stop the flow of molecules through the needles. In a preferred embodiment, flow is controlled by using a rate-limiting membrane as a "valve."

The microneedle devices can further include a flowmeter or other means to monitor flow through the microneedles and to coordinate use of the pumps and valves.

### f. Sensors

The sensing/analyzing function can occur in the microneedle or body of the device (e.g., in the fluid collection chamber), or external to the device following removal of a sample. The sensing device can be in or attached to one or more microneedles, integrated into the substrate, or within or in communication with the fluid collection chamber. Biosensors can be located on the microneedle surface, inside a hollow or porous microneedle, or inside a device in communication with the body tissue via the microneedle (solid, hollow, or porous).

In a preferred embodiment, the microneedle device provides a single-use collection means. In this design, the microneedle array is used to extract a single measurement and then is detached from a reusable base portion, if any, and disposed.

In one embodiment, the microneedle device is adapted to work with "laboratory-on-a-chip" devices, such as made by Caliper Technologies, Palo Alto, California (*see, e.g.,* U.S. Patent No. 5,852,495 to Parce and U.S. Patent No. 5,876.675 to Kennedy); Aclara Biosciences. Hayward, California (*see, e.g.,* U.S. Patent No. 5,858,1 88 to Soane et al. and U.S. Patent No. 5,883,211 to Sassi et al.); and Nanogen, San Diego, California (*see, e.g.,* U.S. Patent Nos. 5,605,662 and 5,632,957 to Heller et al.).

### (i). Types of Sensors

Useful sensors may include sensors of pressure, temperature, chemicals, pH, and/or electro-magnetic fields. These microneedle biosensors can include four classes of principal transducers: potentiometric, amperometric, optical, and physiochemical. An amperometric sensor monitors currents generated when electrons are exchanged between a biological system and an electrode. Blood glucose sensors frequently are of this type.

Sensing information or signals can be transferred optically (e.g., refractive index) or electrically (e.g., measuring changes in electrical impedance, resistance, current, voltage, or combination thereof). For example, it may be useful to measure a change in the resistance of tissue to an electrical current, wherein different resistances are calibrated to signal that withdrawal has been completed.

In a preferred embodiment, the microneedle device may include an integrated sensor, such as a chemical sensor, biosensor, or other measurement system to form a complete extraction/ measurement system. The unit can be constructed to function as a closed loop drug delivery unit, including drug delivery means, analyte recovery means, sensing means to measure the analyte, and control means to provide a signal to the drug delivery means. In a preferred embodiment, the unit would include subunits to withdraw fluid and calculate the concentration of glucose, for example, to determine the amount of insulin needed and deliver that amount of insulin.

The device can include means for assaying the amount of analyte extracted. For example, an assay method that results in a color change could be used. The change in color could be detected using a light beam that enters into a disposable collection chamber through a window on top. The analyte may also be detected in the chamber through the use of an enzyme electrode or biosensor. The analyte sensing system can include enzymes that react with the analyte of interest and either electrochemical or optical transducers that measure the content of reaction. Examples of such enzymes are glucose oxidase and glucose dehydrogenase.

An example of an enzyme electrode for glucose is a screen-printed electrode on the surface of which is immobilized glucose oxidase and an electron mediator such as ferrocene or its derivatives. Electrons generated by the oxidation of glucose are transferred from glucose oxidase to the electrode via the mediator. Thus, the concentration of glucose in the analyte solution is proportional to the current generated. Yet another detection mechanism may be used based on near-infrared spectroscopy. In this method, concentration of extracted glucose in a gel is detected by the absorption of the near-infrared light that passes through the chamber through two windows.

### (ii). Microneedle as Sensing Element

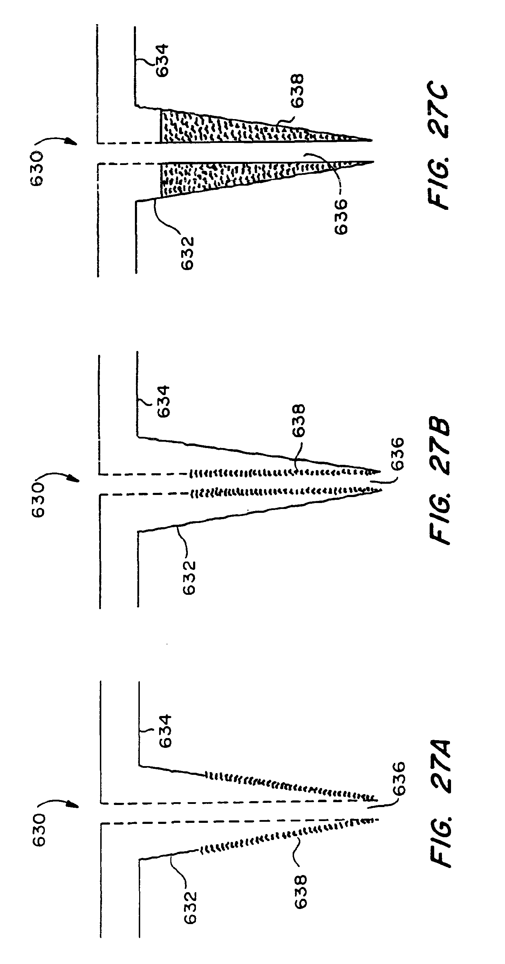

In one embodiment the microneedle is adapted to be the sensing element. For example, an assay material, such as glucose oxidases, can be (i) coated onto the external surface of hollow or solid microneedles, (ii) distributed within the pores of porous microneedles, or (iii) line or fill the bore(s) of hollow microneedles. See Figure 27 which illustrates a cross sectional view of a preferred embodiment of a microneedle device **630** including microneedle **632.** attached to substrate **634** and having hollow bore **636.** In Figure 27a, sensor material **638** is coated on the external surface of microneedle **632.** In Figure 27b, sensor material **638** is coated on the internal surface of hollow bore **636.** In Figure 27c, sensor material **638** is located within the pores of a porous microneedle **632.** Solid microneedles can also hold or contain sensor material as in Figure 27a, or as in Figure 27c if the microneedles are porous. These various microneedle types and sensors can be used in different combinations within a device array.

In this embodiment, the assay material contacts the analyte for which it is selective and undergoes a change, such as an oxidation reaction. This change is communicated, either directly or indirectly, to the user. For example, change may be indicated optically (change in color or refractive index) or electrically. Alternatively, the change may induce a shift in pH that can be measured and communicated using conventional techniques. In more complex embodiments, the analyte may be adsorbed to the microneedle or a coating thereon, such that mechanical sensing can be used, for example, by measuring vibration changes caused by the adsorption.

The microneedle may function as a conduit for fluids, solutes, electric charge, light, or other materials. In one embodiment, hollow microneedles can be filled with a substance, such as a gel, that has a sensing functionality associated with it. In an application for sensing based on binding to a substrate or reaction mediated by an enzyme, the substrate or enzyme can be immobilized in the needle interior, which would be especially useful in a porous needle to create an integral needle/sensor.

### (iii). Sensor Electronics

In a preferred embodiment, the sensors are selectively in communication with an electronics package. The electronics package typically includes a power source (e.g., a battery), as well as electronic hardware and software for the transduction storage, transmission and display of measured values. The electronics package can be selectively fixed to the microneedle device, for example, so that the electronics package can be reused with a new, disposable microneedle device. The electronics package can include a mechanism for wireless or wire-based transmission of measured values to a remote device for analysis and/or display. The electronics also may include mathematical manipulation of the sensed data, for example, to average measured values or eliminate outlying datapoints so as to provide more useful measurements.

The electronics package also can include software and hardware to initiate or automate the sensing and analysis processes. It may be desirable to design a microneedle device capable of taking multiple measurements to withdraw/sense samples on a preprogrammed schedule (e.g., periodic or random), for example, to monitor the blood plasma levels of an illegal drug. Such a device could be adapted for use with a transmitter designed to lock onto the wrist or ankle of a drug offender or other probationer. The device would then be able to randomly test the wearer without his knowledge in order to assess his compliance with orders to abstain from drug or alcohol use.

In an alternative embodiment, the electronics package includes a device for controlling the withdrawal or sensing process. For example, sensing could be activated based on elapsed time, body temperature, or in response to an external trigger (e.g., motion sensor or push-button). The electronics package, for example, could provide substantially instantaneous readings of glucose levels by depressing a button, or in a microneedle device (e.g., adapted to be worn for an extended period of time), the electronics could it could display a summary of intermittent glucose measurements taken automatically throughout the day. Such a microneedle device would be highly useful to diabetic patients to routinely monitor their blood glucose levels following insulin dosing, between or following meals, and at other times throughout the day.

Wave guides can be incorporated into the microneedle device to direct light to a specific location, or for detection, for example, using means such as a pH dye for color evaluation. Similarly, heat, electricity, light or other energy forms may be precisely transmitted to directly stimulate, damage, or heal a specific tissue or intermediary (e.g.. tattoo removal for dark skinned persons), or diagnostic purposes, such as measurement of blood glucose based on IR spectra or by chromatographic means, measuring a color change in the presence of immobilized glucose oxidase in combination with an appropriate substrate.

### (iv). Single Unit Multiple Sensing

A single microneedle device can be designed to provide multiple, preferably sequential, measurements. In a preferred embodiment, the device includes a plurality of discrete fluid collection chambers, each of which is in communication with a defined subset of microneedles in an array of microneedles. The sensing unit of the device can sense each of the collection chambers independently. The sensing can be triggered manually or device electronics can be preprogrammed to automatically trigger, for example at specific time intervals. The device can include a means for storing, displaying, or transmitting the multiple measured values as needed.

### (v). Sensor Calibration

Calibration of the sensor can be accomplished using the concentration of a second analyte or the same analyte measured by another means. The primary analyte can be normalized, lowering extraction to extraction and site to site variability, by the concentration of the second analyte or same analyte from a separate measurement. Normalization may be a linear or non-linear relationship.

In a preferred embodiment for glucose sensing, a reusable sensor, which assays glucose concentration in interstitial fluid, can be calibrated daily by correlating interstitial fluid glucose values with values obtained from glucose measurements obtained from blood, similar to the approach used by Cygnus Inc.'s "Gluco-Watch."

### g. Attachment Features

A collar or flange also can be provided with the device, for example, around the periphery of the substrate or the base. It preferably is attached to the device, but alternatively can be formed as an integral part of the substrate, for example by forming microneedles only near the center of an "oversized" substrate. The collar can also emanate from other parts of the device. The collar can provide an interface to attach the microneedle array to the rest of the device, and can facilitate handling of the smaller devices.

In a preferred embodiment, the microneedle device includes an adhesive to temporarily secure the device to the surface of the biologist barrier. The adhesive can be essentially anywhere on the device to facilitate contact with the biological barrier. For example, the adhesive can be on the surface of the collar (same side as microneedles), on the surface of the substrate between the microneedles (near the base of the microneedles), or a combination thereof.

The device can be integrated into an adhesive patch or an elastic band, which may be worn, for example, around the user's arm or ankle. The arm band can be conveniently worn by a patient for drug delivery, sampling of biological fluids, or both over a prolonged period of time, such as several hours.

Care must be taken so that any adhesive agent does not plug the bores of hollow microneedles. For example, the adhesive agent can be applied in a liquid solution by flooding the top of the substrate below the tips of the microneedles, such as from the side of an array of microneedles, or by using a three-dimensional printing process. The solvent can then be evaporated from the adhesive agent solution, thereby precipitating or gelling the adhesive agent to yield a tacky surface. An alternate method of keeping the tips free of an applied adhesive agent is to choose materials of construction having a hydrophobicity or hydrophilicity to control the wetting of the surface to the microneedle tips.

Where a vacuum is used to induce fluid flow into the device, it may be necessary to provide means for creating a gas-tight seal around the edge of the device so that air is not drawn in through the microneedles at the biological barrier hole/microneedle shaft periphery. The attachment collar or adhesive material can be adapted to provide this function using techniques common to the those skilled in the art. One example is to design the attachment collar to function as a "suction-cup" and wet the interface of the skin and attachment collar with water or another physiologically acceptable sealant.

### h. Transdermal Microneedle Device

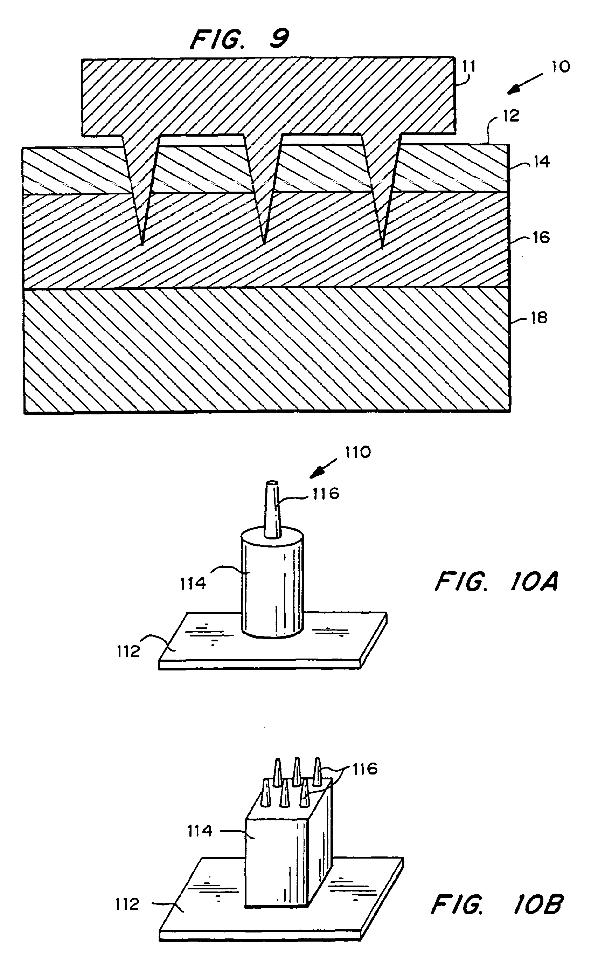

Figure 9 is a side elevational view of a schematic of an embodiment of the microneedle device inserted into undeformed skin. The device **10** includes an upper portion or substrate **11** from which a plurality of microneedles **12** protrude. The height of the upper portion **11** is between about 1 µm and 1 cm, and the width of the upper portion its between about I mm and 10 cm. The upper portion **11** of the device can be solid or hollow, and may include multiple compartments. In a preferred embodiment for drug delivery, the upper portion **11** contains one or more drugs to be delivered. It is also preferred that the upper portion include one or more sensors and/or an apparatus (e.g., pump or electrode) to drive (provide/direct the force) transport of the drug or other molecules.

The height (or length) of the microneedles **12** generally is between about 1 µm and 1 mm. The diameter and length both affect pain as well as functional properties of the needles. In one embodiment for transdermal applications, the "insertion depth" of the microneedles **12** is less than about 100 µm, more preferably about 30 µm, so that insertion of the microneedles **12** into the skin through the stratum corneum **14** does not penetrate through the epidermis **16** into the dermis **18** (as described below), thereby avoiding contacting nerves and reducing the potential for causing pain. In such applications, the actual length of the microneedles may be longer, since the portion of the microneedles distal the tip may not be inserted into the skin; the uninserted length depends on the particular device design and configuration. The actual (overall) height or length of microneedles **12** should be equal to the insertion depth plus the uninserted length. Other embodiments using sufficiently small microneedles may penetrate into the dermis without causing pain.

The diameter of each microneedle **12** generally is between about 10 nm and 1 mm, and preferably leaves a residual hole (following microneedle insertion and withdrawal) of less than about 1 µm, to avoid making a hole which would allow bacteria to enter the penetration wound. The actual microneedle diameter should be larger than 1 µm, since the hole likely will contract following withdrawal of the microneedle. The diameter of microneedle **12** more preferably is between about 1 µm and 100 µm. Larger diameter and longer microneedles are acceptable, so long as the microneedle can penetrate the biological barrier to the desired depth and the hole remaining in the skin or other tissue following withdrawal of the microneedle is sufficiently small, preferably small enough to exclude bacterial entry. The microneedles **12** can be solid or porous, and can include one or more bores connected to upper portion **11.**

### i. Microneedle Penetration Enhancing Features

In a preferred embodiment, the microneedle devices include features which improve penetration of the microneedles into deformable, elastic biological barriers, such as human skin. As used herein, "improved penetration" refers to providing more uniform, better controlled insertion of microneedles, for example, as compared for example, as compared to manual insertion of a microneedle or array of microneedles into skin without compensation (in the device design or site preparation or both) for deformation of the skin at the intended site of insertion. This is particularly critical for devices which include a three dimensional array of the microneedles. These features typically function to (1) adapt to the deformation. (2) limit the deformation, and/or (3) utilize alternate ways of creating the holes in the biological barrier for the microneedles to enter. These penetration enhancing techniques generally can be used with solid, porous, or hollow microneedles. Examples of penetration enhancing features include the following:

### (i) Microneedle Extensions

In a preferred embodiment for adapting the device to the elasticity of skin, the microneedles of the device include extensions, also called protrusion enhancers. One solution to enhance microneedle penetration would be to make the microneedles much longer. However, very tall, small diameter microneedles would tend to be structurally fragile. Therefore, a relatively wider, yet tall base is provided between the substrate and the microneedle to increase the length while maintaining structural integrity of the microneedles. The extensions can be fabricated using the same microfabrication techniques described herein or other conventional fabrication techniques, and typically are made integrally with the substrate and microneedles. The extensions can be of any cross-sectional shape, are typically between about 500 µm and 10 mm in height, and generally are at least about 200 µm in diameter for a single needle/single extension configuration. A single extension can support one or more microneedles.

The extensions also can be designed to function as a penetration stop or limiter, to limit the depth of microneedle penetration.

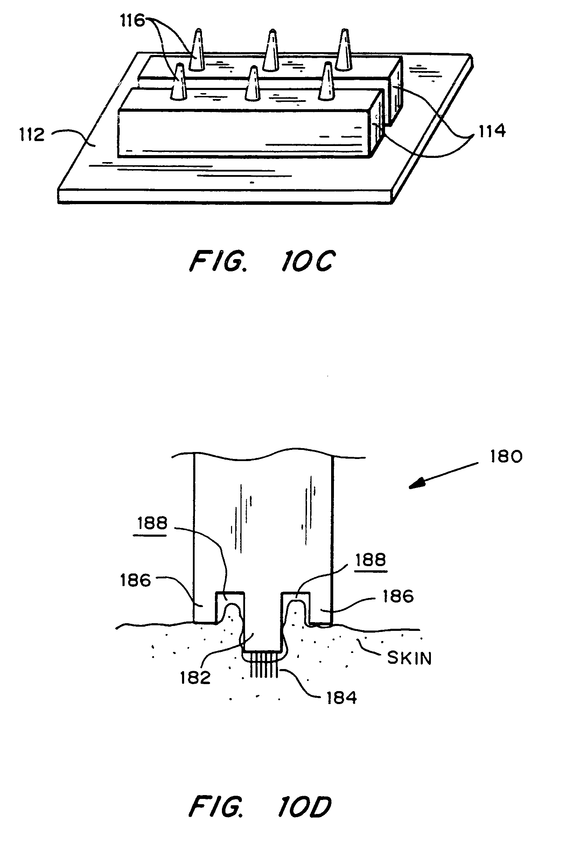

Figures 10a-c illustrate several non-limiting embodiments of the extensions. Device **110** includes substrate **112,** extension **114,** and microneedle(s) **116.** The figures show a single needle/single extension configuration (10a), a multiple needle/single extension configuration (10b), and a multiple needle/multiple extension configuration (10c). The microneedle devices can include arrays of microneedles and extensions in essentially any combination and number.

Figure 10d illustrates an embodiment of a microneedle device **180** having extension **182** terminating in microneedle array **184.** The device also includes shoulder **186** and key (cutout) **188,** which serve to control stretching of the skin at the site of microneedle insertion to enhance penetration. The skin stretches in key **188** as microneedle device **180** is pushed in, while shoulder **186** simultaneously limits the area of skin being stretched.

### (ii). Optimized Microneedle Spacing

In another embodiment to adapt to the deformability of skin, the microneedle device includes arrays of microneedles having spaces between the microneedles or between arrays of microneedles, wherein the spacings permit areas of the skin to fill the spaces in order to enhance microneedle contact with adjacent areas of the skin. The spacing is designed to adapt to the skin's radius of curvature to overcome the penetration problem.

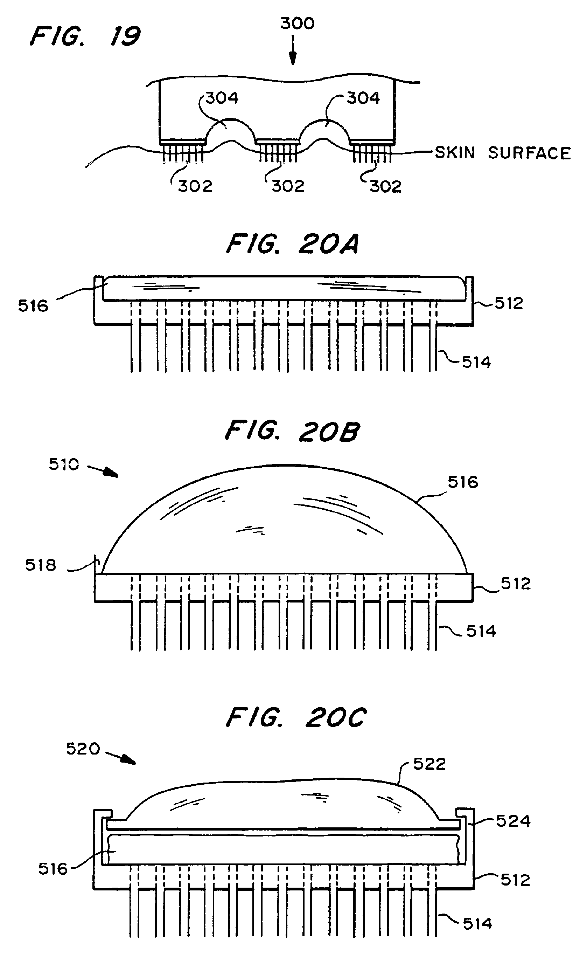

Figure 19 illustrates a microneedle device **300** having arrays of microneedles **302** with spaces **304** between the arrays. Spaces **304** have a size between about one and ten times, preferably between about one and two times, the size of the arrays **302.** This spacing effect can be achieved using some configurations of the microneedle extensions described above.

### (iii). Linear and Curved Arrays of Microneedled

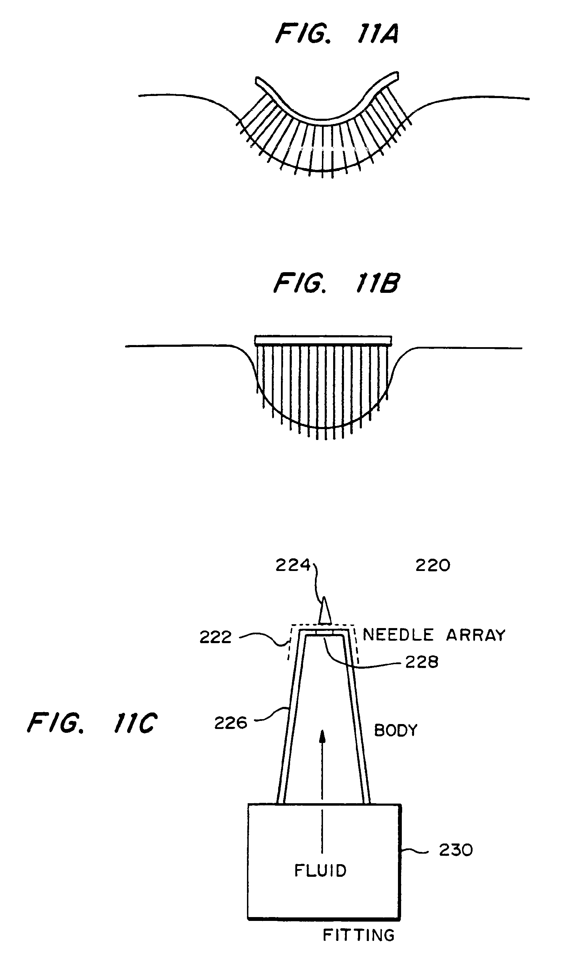

In another embodiment for adapting the device to the elasticity of skin, the microneedles are provided in a curved three dimensional array. For example, the microneedle device can have a rigid substrate which forms a curved, rather than planar, surface. The substrate can be, for example, hemispherical or elliptical. The device, by presenting the needles with a curved surface, can provide improved uniformity of penetration among the microneedles of an array, as illustrated in Figure 11a. This same effect also can be achieved by varying the height of the microneedles in the array having a planar substrate, as shown in Figure 11b.

In a preferred embodiment, two dimensional (i.e. linear) arrays are used. A preferred embodiment of a linear array microneedle device is shown in Figure 11c. Device **220** includes substrate **222.** which is formed into a U-shape, and an array of microneedles **224** at the apex of substrate **222.** The substrate/microneedle portion is mounted over the end of a slotted holder **226.** Molecules for transport through the microneedles **224** flow through one or more slots **228** positioned at the end of the slotted **holder 226.** The device also includes a fitting portion **230** which is integral with or attached to the slotted holder **226**. The fitting portion **230** can be, for example, a female Luer lock for attachment to a conventional syringe. The use of a linear array such as in this device readily deforms the skin at a sharp radius over the tips of the microneedles **224,** greatly facilitating their penetration into the skin.

In a variation of this embodiment, several linear arrays of microneedles can be combined onto a single fitting portion or holder in order to increase the area of injection. By spacing the arrays on the holder widely enough, the correct skin deformation can be maintained. The spacing is selected to be sufficient to allow the skin or other barrier to reach a relaxed state in the region between the arrays, thus facilitating the correct deformation over each needle array in a manner independent of other arrays.

These microneedle devices are fabricated using or adapting the same microfabrication techniques described herein. In one embodiment, microneedle arrays can be fabricated on flexible substrates, which then are mounted onto rigid spherical, cylindrical, or elliptical surfaces.

### (iv). Flexible Substrates

The substrate also can be flexible so that it deforms with the skin or other barrier upon application of the microneedle array.

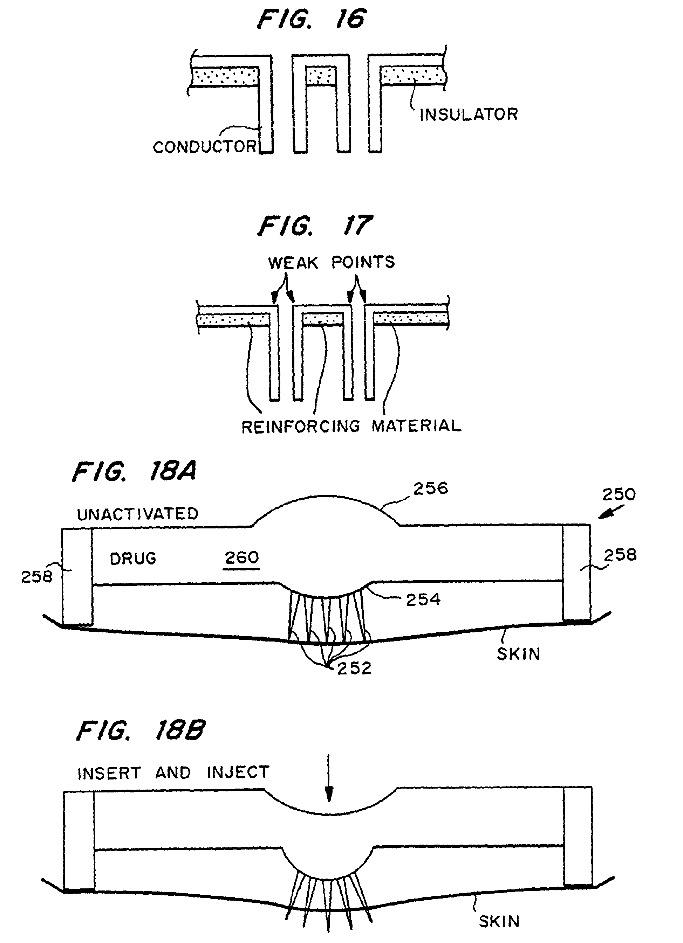

Figures 18a-b illustrate a preferred embodiment of a microneedle device having a flexible substrate. In this case, device **250** include microneedles **252.** which are fabricated on a flexible substrate **254.** The substrate is laminated to a preformed membrane bubble **256,** such as those which are used as a membrane switch. This laminated structure is attached to holder **258.**

The molecules to be delivered, e.g., drug, are contained in a chamber **260** formed between the membrane bubble **256** and the substrate **254.** The molecules are sealed inside the chamber either at the laminating step or are filled after lamination. for example, by injection through the membrane. A sponge or similar device (not shown) may be incorporated into the chamber **260** to contain the molecules before insertion and delivery. The sponge device is compressed by the bubble membrane as it turns to its downward position (*see* Figure 18b).

In operation, the device **250** is pressed against the skin, wherein the holder **258** positions the microneedles **252** against the skin, as shown in Figure 18a. Then, force is applied to the membrane bubble **256,** flopping it through its transition state to the down position. This action applies pressure so the molecules in the chamber **260,** expanding the substrate **254** of the microneedle array and pushing the microneedles **252** through the skin. Subsequently, the pressure forces the molecules through the microneedles **252** and into the skin. It The change in pressure upon the membrane bubble must occur faster and be larger than can be relieved by the flow of molecules through the microneedles, in order to achieve effective delivery.

In an alternative embodiment, the chamber **260** may include an intermediate septum or equivalent divider (not shown) which can be ruptured or moved to permit the molecules to flow through the microneedles **252.** For example, the user applies pressure to the membrane bubble, which pressure first forces the microneedles into the at shown in Figure 18b, and then ruptures the septum to deliver drugs through the needle and into the skin.

### (v). High Velocity Insertion

In another embodiment, the microneedles are applied to the skin surface at an increased velocity, thereby reducing the time available for the stratum corneum and underlying tissues to deform from contact with the tips of the microneedles. The insertion can be by forcing the microneedles into the skin, forcing the skin into the microneedles, or a combination thereof. This more rapid microneedle/skin contact can occur, for example, by releasing a compressed spring or other elastic device that pushes or pulls the microneedles, or by a rapid burst release of compressed gas against the back of the substrate. Alternatively, the skin could be forced by rapidly pulling a vacuum or pushing the skin from the sides (pinching) at the site of microneedle administration to cause the skin to be pulled up against an array of microneedles. Various combinations of these mechanisms can be used together, and typically are integrated into the microneedle device.

### (vi). Limitinig Biological Barrier Elasticity

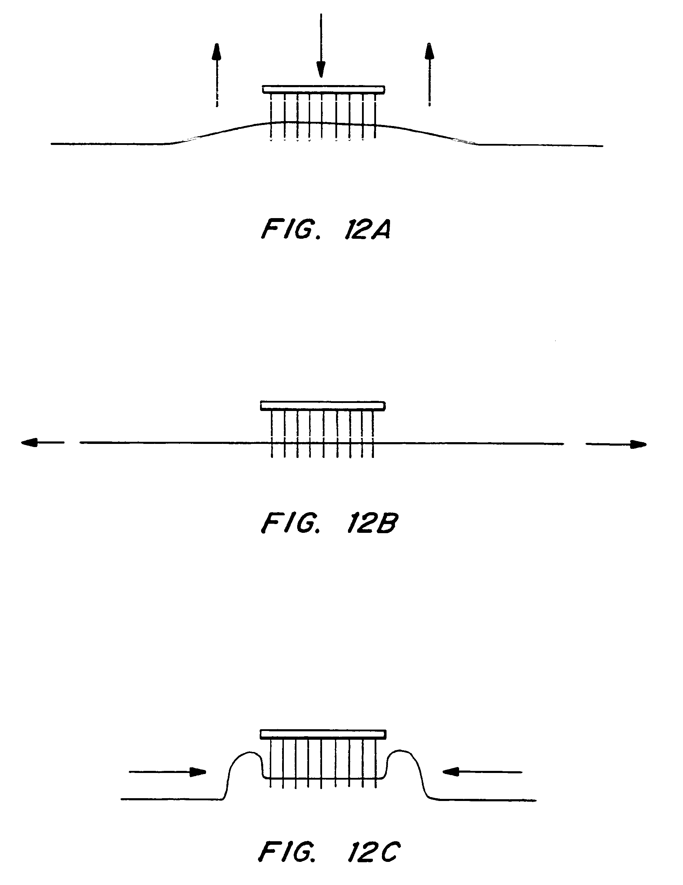

In preferred embodiments for limiting the elasticity/deformation of tissue, the microneedle device includes features suitable for stretching, pulling, or pinching the tissue, particularly skin, to present a more rigid surface in the area to which the microneedles are applied (i.e. penetrate) as illustrated in Figures 12a-c.

It may also be useful to cool the stratum corneum to reduce its elasticity and/or to apply chemicals to the skin surface to "tighten" the skin and induce its elasticity. For example, the skin surface may be cooled by directing a flow of cold gas through the microneedles onto the skin surface. The flow of cold gas can be generated, for example, from a liquefied gas source such as nitrogen, carbon dioxide, or a refrigerant such as FREON^{™}. The skin also can be cooled by contact with a cooling (e.g., refrigerated) element, such as a plate, or the microneedles themselves can function as the cooling element. The cooling element should provide sufficient local cooling of the stratum corneum so as to stiffen the stratum corneum in the vicinity of each needle sufficiently to enhance penetration. These cooling means can be used independently or in combination with chemical means to tighten the skin. Examples of chemical means include biocompatible organic solvents, such as isopropanol or acetone, which tend to dry out and stiffen the skin surface, or such astringent chemicals that can be topically applied to the skin before microneedle insertion.

### (a). suction

Suction can be used to hold the skin in place during the insertion of the microneedle, limiting the deflection and deformation of skin in contact with the tips of the microneedles. Suction also can be used to bring skin in contact with stationary microneedles, and if sufficiently great, can cause the skin to be pierced by the microneedles. The suction may also enhance systemic delivery of drug by increasing blood flow in the area of administration via the microneedles, or may enhance withdrawal of interstitial fluid or blood for analysis/sensing. The suction typically is induced by creating a vacuum on the skin at the site of microneedle application. The vacuum induced is between about 10 and 2000 mm Hg, preferably between about 50 and 300 mm Hg, at the site of application. The suction typically can occur before, simultaneously with, or following insertion of the microneedles.

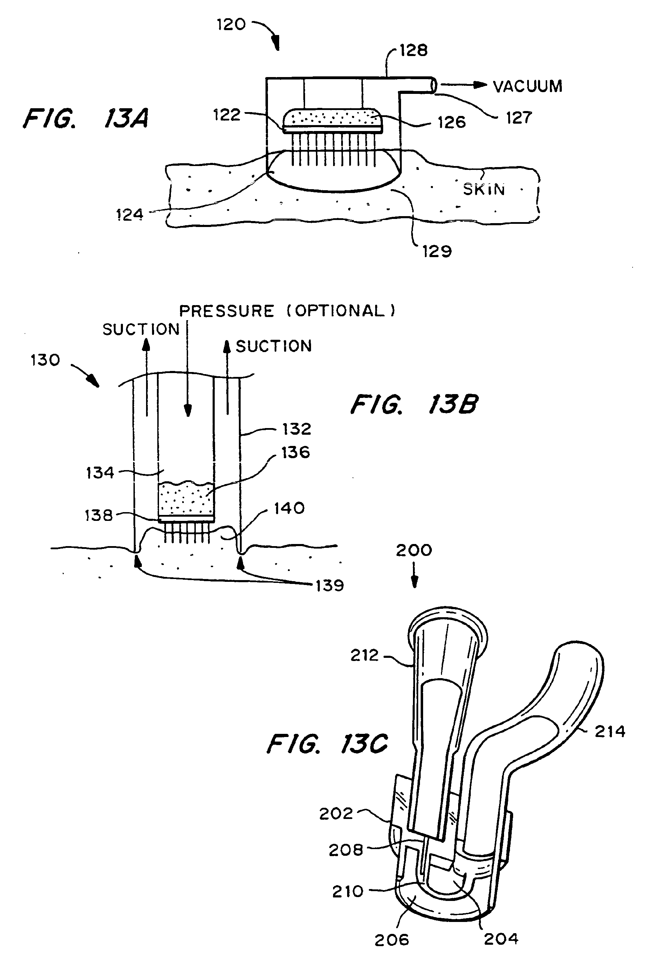

Examples of microneedle devices adapted to create a vacuum are shown in Figures 13a-c. Figure 13a shows device **120** which includes substrate **122,** microneedle array **124.** and reservoir 126. which is partially enclosed by outer chamber **128.** Outer chamber **128** includes a port **127** through which air or other fluids within the chamber are withdrawn following, and/or before, contact of the opening rim **129** to the skin surface. The outer chamber **128** can be made of flexible or rigid material, and generally is gas impermeable. A source of vaccum, e.g., a vacuum pump, typically is adapted to be in fluid communication with port **127**. A micro-battery-powered vacuum pump can be integrated into the device to provide this function. In another embodiment, device **128** can be evacuated, so that when triggered, the skin at the site of administration is exposed to the vacuum and is pulled up into the device. This embodiment effects a short pulse of vacuum, without requiring extra equipment. In another embodiment, a tool can be used to mechanically create the necessary vacuum upon manual activation.

Figure 13b is a variation of the device shown in Figure 13a. wherein device **130** includes two coaxial cylinders, outer cylinder **132** and inner cylinder **134.** Vacuum is induced in the space between the cylinders. Inner cylinder **134** terminates with reservoir **136,** substrate **138,** and microneedles **140.** Outer cylinder **132** and its rim **139** function as the outer chamber **128** and opening rim **129** in Figure 13a. Optionally, an overpressure can be applied in the inner cylinder **134** to facilitate flow. e.g. of drug, from reservoir **136** through the microneedles **140.** The device can be designed to have the inner cylinder **134** and microneedles **140** in a position fixed or movable with respect to the outer cylinder **132,** as appropriate. In other embodiments, the device can have a non-cylindrical shape.

A preferred embodiment of a suction device for use with a microneedle array is shown in Figure 13c. The suction device **200** includes body portion **202** having an inner vacuum ring **204** and an outer vacuum ring **206.** separated by a ring-shaped microneedle array (not shown). The use of coaxial suction rings provides a uniform deformation force on the skin. The amount of deformation can be controlled by varying the absolute and relative sizes of the annular vacuum rings, and by controlling the amount of vacuum pulled, for example, by a suction pump. The relative height of the inner and outer rings, and their height relative to the microneedle array, can be varied to change the skin deformation and consequent pressure onto the microneedle array. In the device shown, molecules, typically as a fluid, are delivered through a port **208** in the annular ring holding the microneedles, and to the whole ring-shaped array through a circular fluidics channel **210** adjacent the substrate of the microneedles. The device shown includes a female Luer lock **212** for attachment to a conventional syringe, as well as tubing section **214** that can be used to connect the device to a vacuum pump of some kind, either manually or power driven.

### (b). stretching

Stretching can also be used to limit the deflection and deformation of skin in contact with the tips of the microneedles. The stretching can be effected by a separate device or incorporated as a feature of the microneedle device itself.

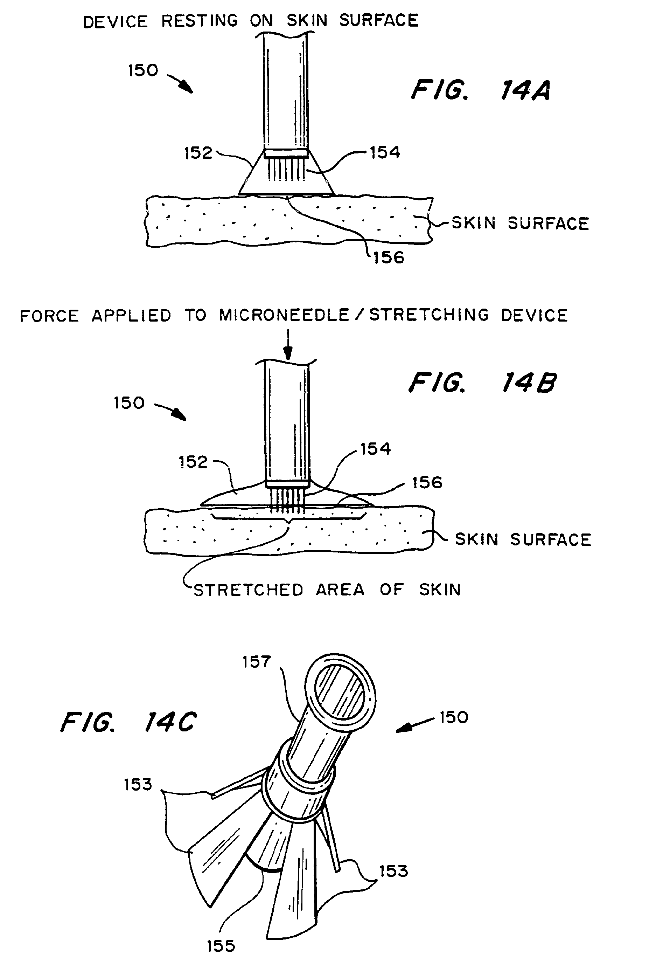

Figures 14a-b shows one embodiment of a microneedle device that includes a stretching component. Microneedle device **150** includes flexible stretching cone **152.** which includes circular outer rim **156** and surrounds microneedles **154.** Figure 14a shows device **150** with the stretching cone **152** in its normal, relaxed form and shape (i.e. no net forces applied to it) resting on the skin surface, with the tips of microneedles **154** terminating inside stretching cone **152.** Figure 14b shows device **150** following application of pressure (e.g., manual pressure) on device **150** toward the skin. As the force is applied, outer rim **156** begins to "flatten" and frictionally engages the skin surface, stretching the skin away from and perpendicular to the central axis of the stretching cone **152,** while the microneedles **154** move into contact with and then penetrate the skin. The outer rim **156** should be roughened, knurled, have teeth, or be formed of or coated with a sticky or non-slippery material so as to provide the necessary engagement between the surfaces. Examples of suitable materials include rubbers and synthetic polymers.

In another embodiment, the device includes an aperture or other means of allowing gas trapped between the barrier and the device to escape. Examples of such apertures include one or more vent holes or slits, for example, through the side of the stretching cone **152.**

A preferred embodiment is shown in Figure 14c, wherein stretching cone **152** is replaced with a plurality of. e.g., four, hinged stretching elements **153.** The stretching elements stretch the skin laterally as device **150** is pressed down against the skin. The skin deflection is determined by the length of the stretcher element 153 relative to the length of the central cylinder **155.** Like outer rim **156,** the ends of the stretching elements **153** (distal the hinge end) preferably are roughened or knurled, have teeth, or are formed of or coated with a sticky or non-slippery material so as to control the friction between the skin and the stretching elements **153,** thereby providing an additional means of controlling the stretching force. The device shown in Figure 14c is connected to a standard female Luer lock **157,** for attachment to a standard syringe. In a preferred embodiment, microneedles **154** are aligned in columns and rows substantially parallel to the stretching element **153,** so as to optimize the stretching force for effective microneedle penetration.

In another embodiment, stretching of the skin is accomplished using a separate ring device, which can be pressed against the skin and then concentrically expanded to stretch the skin within the ring. For example, the ring device can be a rubber-coated metal band (having diameter x), which is compressed into a coil (having a diameter less than x). The coiled ring is then pressed against the skin and allowed to uncoil (i.e. expand to diameter x again), while frictionally engaging the skin, thereby stretching it. In a further embodiment, the ring device is configured as an iris that expands to stretch the skin upon mechanical actuation by engaging an actuation lever or rotary motion

### (c). pinching

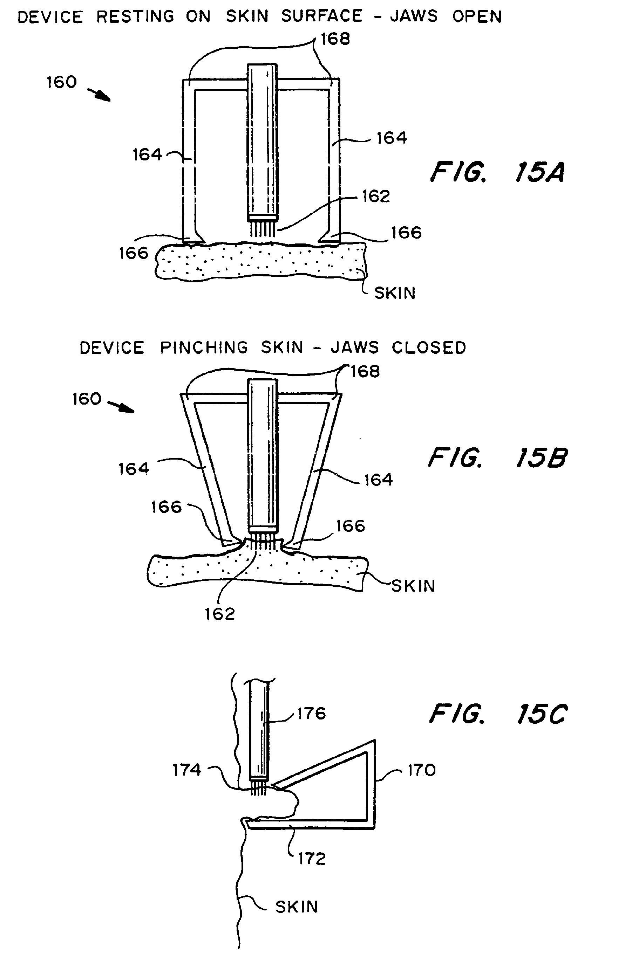

Pinching can also be used to limit the deflection and deformation of skin in contact with the tips of the microneedles. In a preferred embodiment, the microneedle device includes jaws, typically one or more pair, which can be pressed against the skin surface and triggered to close against a segment of skin, as illustrated in Figures 15a-b. The size of the jaw opening can be selected based on the area of skin to be pinched to facilitate penetration of the microneedle array selected for use.

In Figures 15a-b, microneedle device **160** includes a pair of jaws **164** having flex or hinge points **168** and tips **166,** positioned around microneedles **162.** The Figures show the jaws **164** in the open position (Fig. 15a) and in the closed position (Fig. 15b), pinching an area of skin which is drawn against the microneedle **162,** causing the microneedles to penetrate the skin.

An alternative embodiment of the clamping device and method is illustrated in Figure 15c, wherein clamping device **170** is used to pinch the skin and provide a rigid support **172** behind a pinched section of skin, in order to present a surface of limited deformability for insertion of the microneedles **174.** Clamping device **170** can be a separate device from the microneedle device **176.** or the devices can be integrated into a single unit.

### (d) adhesive film assist

The deformation of skin can be reduced by applying a thin, adhesive film over the skin surface at the site of application. The film keeps the skin taut and iimits deformation at the site of microneedle insertion as the microneedles are pushed through the film and into the skin. This is analogous to the use of a surgical tape that is applied to the skin before making a surgical incision.

The adhesive film can include an expandable or shrinkable material that is triggered to change size or shape prior to microneedle application, so as to stretch the skin thereby further limiting its deformability. For example, the material can be water-swellable, and wetted (triggering event) prior to microneedle application. In another example, the material can change form in response to a change in temperature (triggering event).

The adhesive film must be thin enough for the microneedles to penetrate both the film and the stratum corneum. If hollow microneedles are used, then the film should fracture upon penetration without substantial clogging of the hollow bore of the microneedles. Alternatively, the film could be formed of a woven or porous material so that the microneedles substantially penetrate gaps or pores in the adhesive film rather than the fibers or matrix of the film material. Examples of suitable films include ethylene-tetrafluoroethylene (ETFE) copolymer mesh (available from Goodfellow PLC) and porous films such as GORETEXTM (available from William H. Gore, Inc.) coupled with an appropriate acrylic adhesive.

### (vii). Creating Holes for the Microneedles

Another method of improving the penetration of biological barriers with microneedles involves producing holes or pathways in the barrier through which the microneedles can traverse, other than the pathway created solely by forcing the microneedle into barrier. A variety of techniques can be adapted for use with the microneedle devices described herein. For example, U.S. Patent No. 5,885,211 to Eppstein, et al. discloses several methods of selectively removing the stratum corneum to enhance permeability of human skin. Some of these so-called microporation techniques disclosed can be adapted to promote penetration of microneedles into the viable epidermis. In a preferred embodiment, holes are created by vaporizing the stratum corneum, for example, by using a laser or by heating of the tips of the microneedles.

Laser techniques are described, for example, in U.S. patents No. 5,885,211 to Eppstein, et al. and No. 4,775,361 to Jacques et al. The laser, or pulsed light source, preferably is used in conjunction with a dye which substantially absorbs over the emission range of the light. The dye is applied to the skin and the laser or light is focused on the dye, heating it. Then, the stratum corneum adjacent to the dye is heated by conduction, elevating the temperature of tissue-bound water and other vaporizable substances in the selected area above the vaporization point of the water and other vaporizable substances. This vaporization results in degradation of the stratum corneum at one or more select, i.e. pinpoint, areas, through which the microneedles can readily penetrate. One of skill in the art can readily select the appropriate dye, laser (e.g., Helium-Neon) or light source, and parameters of use, based, in part, on the particular microneedle array, drug to be delivered, and/or analyte to sample. In one embodiment, the light is focused, in part, by passing the light through the internal bore of a hollow microneedle.

Thermal ablation of the stratum corneum can be achieved by radiant heating (using a light/laser as described above) or by using conductive heating. For example. U.S. Patent No. 5,885,211 to Eppstein, et al. describes contacting human skin with a heat source (conductive heating) ablate the stratum corneum. The microneedle devices described herein can be applied to the ablated skin and/or can be configured to provide the ablation of the stratum corneum,in particularly by heating the microneedles to serve as the heat source which contacts the skin. Heating of the microneedles can be accomplished, for example, by (1) contacting the needles with an ohmic heating element, (2) providing microneedles formed of or coated with a conductive material, through which a modulated electrical current is passed to induce resistive heating of the microneedles, or (3) providing microneedles positioned in a modulatable alternating magnetic field of an excitation coil such that energizing the excitation coil with alternating current produces eddy currents sufficient to heat the microneedles by internal ohmic losses. The microneedles should be able to rapidly heat the skin surface at select spots to above 100 °C, preferably above 123 °C. to induce flash vaporization of the water content of the stratum corneum, as described in U.S. Patent No. 4,775,361 to Jacques et al. The heating preferably is done using an on/off cycling technique and/or adjacent cooling to minimize damage to tissues adjacent the target area. In embodiments in which the microneedles are heated, the substrate preferably includes an insulating material, as shown in Figure 16, which illustrates two hollow microneedles and substrate in cross-section.

Other embodiments include applying a jet or focused blast of high pressure fluid to hydraulically puncture the stratum corneum and form a micropore into which the microneedle is inserted. The micropore preferably is slightly larger than the diameter of the microneedle. The microneedle device can be designed to utilize hollow microneedles to direct the jet of high pressure fluid. Various devices and techniques for high velocity introduction of fluids and particles into skin for delivery of drugs or genes are described, for example, in U.S. Patents No. 5,919,159 and No. 5,599,302 to Lilley, et al. (Medi-Ject, Inc.), U.S. Patent No. 5, 899,880 to Bellhouse, et al. (PowderJect Research Ltd.), and U.S. Patent No. 5,865,796 to McCabe (PowderJect Vaccines, Inc.). Creation of the holes and penetration can be enhanced by select cooling/freezing of the surface of the skin. e.g., cryoablation (see U.S. Patent No. 5,147,355 to Friedman, et al.). For example, the microneedle device can be adapted to create a local Joule-Thompson effect (see U.S. Patent No. 5,758,505 to Dobak III, et al.).

In another embodiment, chemical agents, such as certain keratin reducing agents (see U.S. Pat. No. 5,911,223 to Weaver et al.), can be applied at the site of administration to degrade the keratin of the skin's stratum corneum, rendering it more porous. Examples of suitable chemicals include sodium thiosulfate and urea.

### (viii). Lubricated Microneedles

In one embodiment, the microneedles include a lubricating material, such as TEFLON^{™} (polytetrafluoroethylene), coated onto the microneedles. In a preferred embodiment, the lubricating material is incorporated into metal microneedles, for example, by plating the lubricating material with the metal during the manufacture of the microneedles.

### (ix). Vibrating the Microneedles

Essentially all of the microneedle devices and methods described herein can be adapted to vibrate the microneedles and/or the skin to further facilitate penetration. The vibration can be effected to move the microneedles perpendicular and/or parallel to the surface of the biological barrier, and/or at an orientation thereinbetween. The vibration motion can be induced using known techniques, the most common or which is coupling the microneedle or array thereof to a piezoelectric transducer that can provide the vibratory motion. Such a transducer can be bonded directly to the array or can be bonded to a reservoir, thereby utilizing the acoustic transmission properties of the reservoir contents (e.g., an aqueous drug solution) to transmit vibration to the microneedles. Alternatively, electromechanical actuation can be used to vibrate the microneedles, such electromechanical actuation means include miniature motors and speaker coils.

### 3. Methods of Making Microneedle Devices

The microneedle devices are made by microfabrication processes, by creating small mechanical structures in silicon, metal, polymer, and other materials. These microfabrication processes are based on well-established methods used to make integrated circuits, electronic packages and other microelectronic devices, augmented by additional methods used in the field of micromachining. The microneedle devices can have dimensions as small as a few nanometers and can be mass-produced at low per-unit costs.

### a. Microfabrication Processes

Microfabrication processes that may be used in making the microneedles disclosed herein include lithography; etching techniques, such as wet chemical, dry, and photoresist removal; thermal oxidation of silicon; electroplating and electroless plating; diffusion processes, such as boron, phosphorus, arsenic, and antimony diffusion; ion implantation; film deposition, such as evaporation (filament, electron beam, flash, and shadowing and step coverage), sputtering, chemical vapor deposition (CVD), epitaxy (vapor phase, liquid phase, and molecular beam), electroplating, screen printing, lamination, stereolithography, laser machining, and laser ablation (including projection ablation). *See generally* Jaeger, Introduction to Microelectronic Fabrication (Addison-Wesley Publishing Co., Reading MA 1988); Runyan, et al., Semiconductor Integrated Circuit Processing Technology (Addison-Wesley Publishing Co., Reading MA 1990); Proceedings of the IEEE Micro Electro Mechanical Systems Conference 1987-1998*;* Rai-Choudhury, ed., Handbook of Microlithography, Micromachining & Microfabrication (SPIE Optical Engineering Press. Bellingham, WA 1997).

The following methods are preferred for making microneedles.

### (i). Electrochemical Etching of Silicon

In this method, electrochemical etching of solid silicon to polous silicon is used to create extremely fine (on the order of 0.01 µm) silicon networks which can be used as piercing structures. This method uses electrolytic anodization of silicon in aqueous hydrofluoric acid, potentially in combination with light, to etch channels into the silicon. By varying the doping concentration of the silicon wafer to be etched, the electrolytic potential during etching, the incident light intensity, and the electrolyte concentration, control over the ultimate pore structure can be achieved. The material not etched (i.e. the silicon remaining) forms the microneedles. This method has been used to produce irregular needle-type structures measuring tens of nanometers in width.

### (ii). Plasma Etching

This process uses deep plasma etching of silicon to create microneedles with diameters on the order of 0.1 µm or larger. Needles are patterned directly using photolithography, rather than indirectly by controlling the voltage (as in electrochemical etching), thus providing greater control over the final microneedle geometry.

In this process, an appropriate masking material (e.g., metal) is deposited onto a silicon wafer substrate and patterned into dots having the diameter of the desired microneedles. The wafer is then subjected to a carefully controlled plasma based on fluorine/oxygen chemistries to etch very deep, high aspect ratio trenches into the silicon. *See, e.g.,* Jansen, et al., "The Black Silicon Method IV: The Fabrication of Three-Dimensional Structures in Silicon with High Aspect Ratios for Scanning Probe Microscopy and Other Applications." IEEE Proceedings of Micro Electro Mechanical Systems Conference, pp. 88-93 (1995). Those regions protected by the metal mask remain and form the needles. This method is further described in Example 1 below.

### (iii). Electroplating

In this process, a metal layer is first evaporated onto a planar substrate. A layer of photoresist is then deposited onto the metal to form a patterned mold which leaves an exposed-metal region in the shape of needles. By electroplating onto the exposed regions of the metal seed layer, the mold bounded by photoresist can be filled with electroplated material. Finally, the substrate and photoresist mold are removed, leaving the finished microneedle array. The microneedles produced by this process generally have diameters on the order of 1 µm or larger. *See e.g.*, Frazier. et al., "Two dimensional metallic microelectrode arrays for extracellular stimulation and recording of neurons", IEEE Proceedings of the Micro Electro Mechanical Systems Conference, pp. 195-200 (1993).

### (iv). Other Processes

Another method for forming microneedles made of silicon or other materials is to use microfabrication techniques such as photolithography, plasma etching, or laser ablation to make a mold form (A), transferring that mold form to other materials using standard mold transfer techniques, such as embossing or injection molding (B), and reproducing the shape of the original mold form (A) using the newly-created mold (B) to yield the final microneedles (C). Alternatively, the creation of the mold form (A) could be skipped and the mold (B) could be microfabricated directly, which could then be used to create the final microneedles (C).

Another method of forming solid silicon microneedles is by using epitaxial growth on silicon substrates, as is utilized by Containerless Research, Inc. (Evanston, Illinois. USA) for its products.

### b. Hollow or Porous Microneedles

In a preferred embodiment, microneedles are made with pores or other pathways through which material may be transported. The following descriptions outline representative methods for fabricating either porous or hollow microneedles.

### (i). Porous Microneedles

Rather than having a single, well-defined hole down the length of the needle, porous needles are filled with a network of channels or pores which allow conduction of fluid or energy through the needle shaft. It has been shown that by appropriate electrochemical oxidation of silicon, pore arrays with high aspect ratios and a range of different pore size regimes can be formed; these pore regimes are defined as (1) microporous regime with average pore dimensions less than 2 nm, (2) mesoporous regime with average pore sizes of between 2 nm and 50 nm, and (3) macroporous regime with pores greater than 50 nm. The mesoporous and macroporous regimes are expected to be most useful for drug delivery. Two approaches to porous needles are generally available, either (a) the silicon wafer is first made porous and then etched as described above to form needles or (b) solid microneedles are etched and then rendered porous, for example, by means of electrochemical oxidation, such as by anodization of a silicon substrate in a hydrofluoric acid electrolyte. The size distribution of the etched porous structure is highly dependent on several variables, including doping kind and illumination conditions, as detailed in Lehmann. "Porous Silicon--A New Material for MEMS". IEEE Proceedings of the Micro Electro Mechanical Systems Conference, pp. 1-6 (1996). Porous polymer or metallic microneedles can be formed, for example, by micromolding a polymer containing a volatilizable or leachable material, such as a volatile salt, dispersed in the polymer or metal, and then volatilizing or leaching the dispersed material, leaving a porous polymer matrix in the shape of the microneedle.

### (ii). Hollow Microneedles

Three-dimensional arrays of hollow microneedles can be fabricated, for example, using combinations of dry etching processes (Laermer, et al., "Bosch Deep Silicon Etching: Improving Uniformity and Etch Rate for Advanced MEMS Applications," Micro Electro Mechanical Systems, Orlando, Fl, USA, (Jan. 17-21, 1999); Despont et al., "High-Aspect-Ratio, Ultrathick, Negative-Tone Near-UV Photoresist for MEMS", Proc. of IEEE 10th Annual International Workshop on MEMS, Nagoya, Japan, pp. 518-22 (Jan. 26-30, 1997)); micromold creation in lithographically-defined and/or laser ablated polymers and selective sidewall electroplating; or direct micromolding techniques using epoxy mold transfers.

One or more distinct and continuous pathways are created through the interior of microneedles. In a preferred embodiment, the microneedle has a single annular pathway along the center axis of the microneedle. This pathway can be achieved by initially chemically or physically etching the holes in the material and then etching away microneedles around the hole. Alternatively, the microneedles and their holes can be made simultaneously or holes can be etched into existing microneedles. As another option, a microneedle form or mold can be made, then coated, and then etched away, leaving only the outer coating to form a hollow microneedle. Coatings can be formed either by deposition of a film or by oxidation of the silicon microneedles to a specific thickness, followed by removal of the interior silicon. Also, holes from the backside of the wafer to the underside of the hollow needles can be created using a front-to-backside infrared alignment followed by etching from the backside of the wafer.

### (a) silicon microneedles

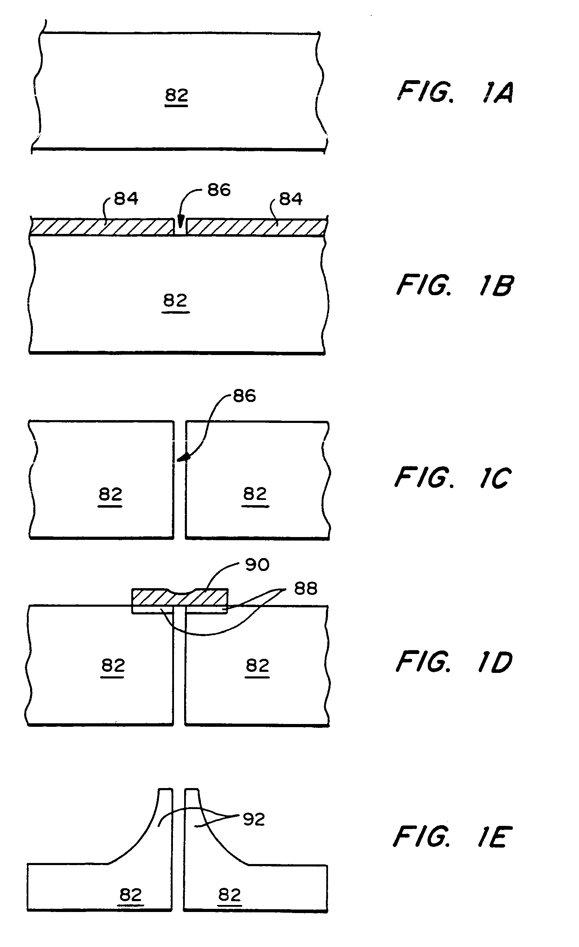

One method for hollow needle fabrication is to replace the solid mask used in the formation of solid needles by a mask that includes a solid shape with one or more interior regions of the solid shape removed. One example is a "donut-shaped" mask. Using this type of mask, interior regions of the needle are etched simultaneously with their side walls. Due to lateral etching of the inner side walls of the needle, this may not produce sufficiently sharp walls. In that case, two plasma etches may be used, one to form the outer walls of the microneedle (i.e.. the 'standard' etch), and one to form the inner hollow core (which is an extremely anisotropic etch, such as in inductively-coupled-plasma "ICP" etch). For example, the ICP etch can be used to form the interior region of the needle followed by a second photolithography step and a standard etch to form the outer walls of the microneedle. Figure 1a represents a silicon wafer **82** with a patterned photoresist layer **84** on top of the wafer **82.** The wafer **82** is anisotropically etched (Figure 1b) to form a cavity **86** through its entire thickness (Figure 1c). The wafer **82** is then coated with a chromium layer **88** followed by a second photoresist layer **90** patterned so as to cover the cavity **86** and form a circular mask for subsequent etching (Figure 1d). The wafer **82** is then etched by a standard etch to form the outer tapered walls **92** of the microneedle (Figure 1e).

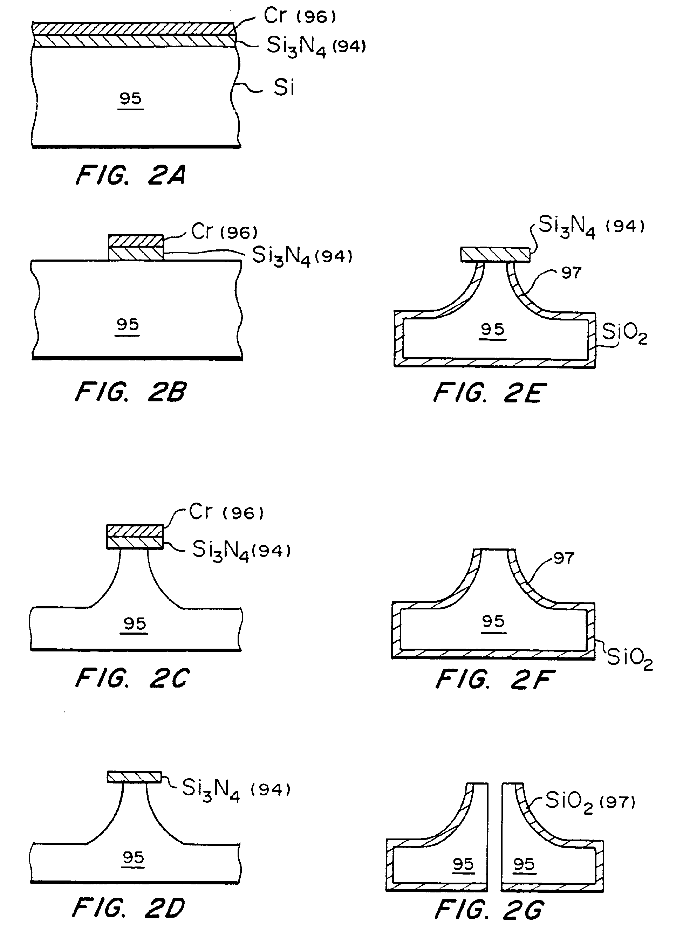

Alternatively, this structure can be achieved by substituting the chromium mask used for the solid microneedles described in Example 1 by a silicon nitride layer **94** on the silicon substrate **95** covered with chromium **96,** deposited as shown in Figure 2a and patterned as shown in Figure 2b. Solid microneedles are then etched as described in Example 1 as shown Figure 2c, the chromium **96** is stripped (Figure 2d), and the silicon **95** is oxidized to form a thin layer of silicon dioxide **97** on all exposed silicon surfaces (Figure 2e). The silicon nitride layer **94** prevents oxidation at the needle tip. The silicon nitride **94** is then stripped (Figure 2f), leaving exposed silicon at the tip of the needle and oxide-covered silicon 97 everywhere else. The needle is then exposed to an ICP plasma which selectively etches the inner sidewalls of the silicon **95** in a highly anisotropic manner to form the interior hole of the needle (Figure 2g).

Another method uses the solid silicon needles described previously as 'forms' around which the actual needle structures are deposited. After deposition, the forms are etched away, yielding the hollow structures. Silica needles or metal needles can be formed using different methods. Silica needles can be formed by creating needle structures similar to the ICP needles described above prior to the oxidation described above. The wafers are then oxidized to a controlled thickness, forming a layer on the shaft of the needle form which will eventually become the hollow microneedle. The silicon nitride is then stripped and the silicon core selectively etched away (e.g., in a wet alkaline solution) to form a hollow silica microneedle.

In a preferred embodiment, an array of hollow silicon microtubes is made using deep reactive ion etching combined with a modified black silicon process in a conventional reactive ion etcher, as described in Example 3 below. First, arrays of circular holes are patterned through photoresist into SiO₂, such as on a silicon wafer. Then the silicon can be etched using deep reactive ion etching (DRIE) in an inductively coupled plasma (ICP) reactor to etch deep vertical holes. The photoresist was then removed. Next, a second photolithography step patterns the remaining SiO₂ layer into circles concentric to the holes, leaving ring shaped oxide masks surrounding the holes. The photoresist is then removed and the silicon wafer again deep silicon etched, such that the holes are etched completely through the wafer (inside the SiO₂ ring) and simultaneously the silicon is etched around the SiO₂ ring leaving a cylinder.

This latter process can be varied to produce hollow, tapered microneedles. After an array of holes is fabricated as described above, the photoresist and SiO₂ layers are replaced with conformal DC sputtered chromium rings. The second ICP etch is replaced with a SF₆/O₂ plasma etch in a reactive ion etcher (RIE), which results in positively sloping outer sidewalls. Henry, et al.. "Micromachined Needles for the Transdermal Delivery of Drugs," Micro Electro Mechanical Systems, Heidelberg, Germany, pp. 494-98 (Jan. 26-29, 1998).

### (b). metal microneedles

Metal needles can be formed by physical vapor deposition of appropriate metal layers on solid needle forms, which can be made of silicon using the techniques described above, or which can be formed using other standard mold techniques such as embossing or injection molding. The metals are selectively removed from the tips of the needles using electropolishing techniques, in which an applied anodle potential in an electrolytic solution will cause dissolution of metals more rapidly at sharp points, due to concentration of electric field lines at the sharp points. Once the underlying silicon needle forms have been exposed at the tips, the silicon is selectively etched away to form hollow metallic needle structures. This process could also be used to make hollow needles made from other materials by depositing a material other than metal on the needle forms and following the procedure described above.

A preferred method of fabricating hollow metal microneedles utilizes micromold plating techniques, which are described as follows and in Examples 4 and 5. In a method for making metal microtubes, which does not require dry silicon etching, a photo-defined mold first is first produced, for example, by spin casting a thick layer, typically 150 µm, of an epoxy (e.g., SU-8) onto a substrate that has been coated with a thin sacrificial layer, typically about 10 to 50 nm. Arrays of cylindrical holes are then photolithographically defined through the epoxy layer, which typically is about 150 µm thick. (Despont, et al.. "High-Aspect-Ratio. Ultrathick. Negative-Tone Near-UV Photoresist for MEMS," Proc. of IEEE 10th Annual International Workshop on MEMS. Nagoya, Japan, pp. 518-22 (Jan. 26-30, 1997)). The diameter of these cylindrical holes defines the outer diameter of the tubes. The upper surface of the substrate, the sacrificial layer, is then partially removed at the bottom of the cylindrical holes in the photoresist. The exact method chosen depends on the choice of substrate. For example, the process has been successfully performed on silicon and glass substrates (in which the upper surface is etched using isotropic wet or dry etching techniques) and copper-clad printed wiring board substrates. In the latter case, the copper laminate is selectively removed using wet etching. Then a seed layer, such as Ti/Cu/Ti (e.g., 30 nm/200 nm/30 nm), is conformally DC sputter-deposited onto the upper surface of the epoxy mold and onto the sidewalls of the cylindrical holes. The seed layer should be electrically isolated from the substrate. Subsequently, one or more electroplatable metals or alloys are electroplated onto the seed layer. Representative suitable metals and alloys include Ni, NiFe, Au, Cu. Cr, Pt, Pd, and Ti. The surrounding epoxy is then removed, leaving microtubes which each have an interior annular hole that extends through the base metal supporting the tubes. The rate and duration of electroplating is controlled in order to define the wall thickness and inner diameter of the microtubes. In one embodiment, this method was used to produce microtubes having a height of between about 150 and 250 µm, an outer diameter of between about 40 and 120 µm, and an inner diameter of between about 30 and 110 µm (i.e., having a wall thickness of 10 µm). In a typical array, the microtubes have a tube center-to-center spacing of about 150 µm, but can vary depending on the desired needle density.

A variation of this method is preferred for forming tapered microneedles. As described above, photolithography yields holes in the epoxy which have vertical sidewalls, such that the resulting shafts of the microneedles are straight, not tapered. This vertical sidewall limitation can be overcome by molding a preexisting 3D structure, i.e., a mold-insert. The subsequent removal of the mold-insert leaves a moid which can be surface plated similarly to the holes produced by photolithography described above.

Alternatively, non-vertical sidewalls can be produced directly in the polymeric mold into which electroplating will take place. For example, conventional photoresists known in the art can be exposed and developed in such as way as to have the surface immediately adjacent to the mask be wider than the other surface. Specialized greyscale photoresists in combination with greyscale masks can accomplish the same effect. Laser-ablated molds can also be made with tapered sidewalls, e.g., by optical adjustment of the beam (in the case of serial hole fabrication) or of the reticle or mold during ablation (in the case of projection ablation).

To form hollow tapered microneedles, the mold-insert is an array of solid silicon microneedles, formed as described in Henry, et al., "Micromachined Needles for the Transdermal Delivery of Drugs," Micro Electro Mechanical Systems, Heidelberg, Germany, Jan. 26-29, pp. 494-98 (1998). First, a layer of a material, such as an epoxy (e.g., SU-8 or a polydimethylsiloxane ("PDMS")), is spin cast onto the array of silicon needles to completely blanket the entire array. The epoxy settles during pre-bake to create a planar surface above the silicon needle tips; the material is then fully pre-baked, photolithographically cross-linked, and post-baked.

The upper surface of the epoxy is then etched away, for example with an O₂/CHF₃ plasma, until the needle tips are exposed, preferably leaving between about 1 and 5 µm of tip protruding from the epoxy. The silicon is then selectively removed, for example by using a SF₆ plasma or a HNO₃/HF solution. The remaining epoxy micromold is the negative of the microneedles and has a small diameter hole where the tip of the microneedle formerly protruded.

After the removal of the silicon, a seed layer, such as Ti-Cu-Ti, is conformally sputter-deposited onto the epoxy micromold. Following the same process sequence described for hollow metal microtubes, one or more electroplatable metals or alloys, such as Ni, NiFe, Au, or Cu, are electroplated onto the seed layer. Finally, the epoxy is removed, for example by using an O₂/CHF₃ plasma, leaving an array of hollow metal microneedles. An advantage of using PDMS in this application is that the micromold can be physically removed from the silicon mold insert by mechanical means, such as peeling, without damaging the silicon mold insert, thus allowing the silicon mold insert to be reused. Furthermore, the electroplated microneedles can be removed from the PDMS mold by mechanical means, for example by peeling, thereby allowing the PDMS to also be reused. In a preferred embodiment, this method is used to produce microneedles having a height of between about 150 and 250 µm, an outer diameter of between about 40 and 120 µm, and an inner diameter of between about 50 and 100 µm. In a typical array, the microtubes have a tube center-to-center spacing of about 150 µm, but can vary depending on the desired needle density. The microneedles are 150 µm in height with a base diameter of 80 µm, a tip diameter of 10 µm, and a needle-to-needle spacing of 150 µm.

### (c) silicon dioxide microneedles

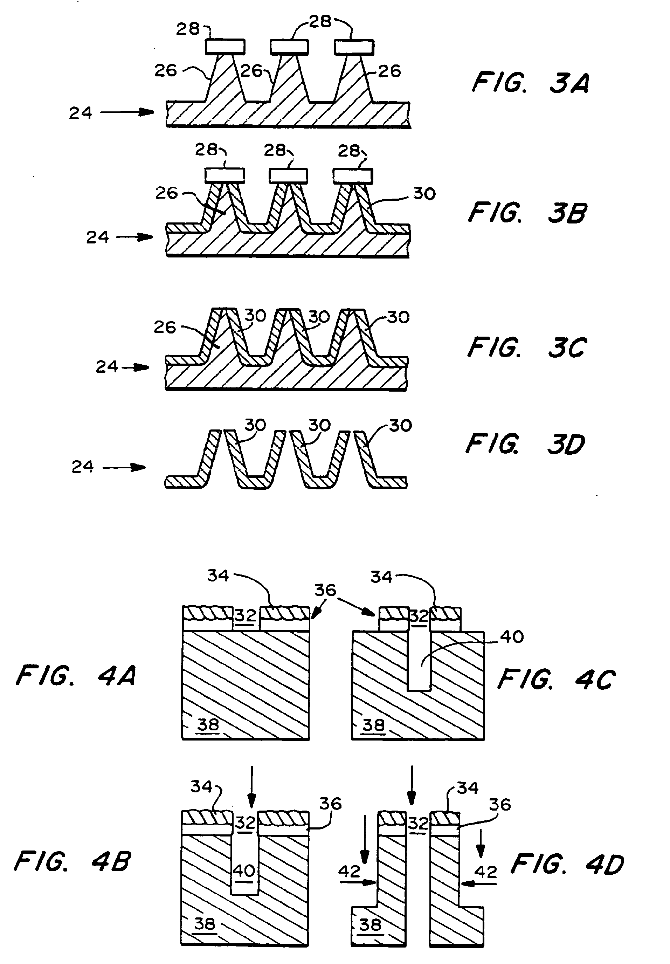

Hollow microneedles formed of silicon dioxide can be made by oxidizing the surface of the silicon microneedle forms (as described above), rather than depositing a metal and then etching away the solid needle forms to leave the hollow silicon dioxide structures. This method is illustrated in Figures 3a-d. Figure 3a shows an array **24** of needle forms **26** with masks **28** on their tips. In Figure 3b. the needle forms **26** have been coated with a layer **30** of metal, silicon dioxide or other material. Figure 3c shows the coated needle forms **26** with the masks **28** removed. Finally, in Figure 3d, the needle forms **26** have been etched away, leaving hollow needles 30 made of metal, silicon dioxide, or other materials.

In one embodiment, hollow, porous, or solid microneedles are provided with longitudinal grooves or other modifications to the exterior surface of the microneedles. Grooves, for example, should be useful in directing the flow of molecules along the outside of microneedles.

### (d). polymer microneedles

In a preferred method, polymeric microneedles are made using microfabricated molds. For example, the epoxy molds can be made as described above and injection molding techniques can be applied to form the microneedles in the molds (Weber, et al., "Micromolding - a powerful tool for the large scale production of precise microstructures", Proc. SPIE - International Soc. Optical Engineer. 2879:156-67 (1996); Schift, et al., "Fabrication of replicated high precision insert elements for micro-optical bench arrangements" Proc. SPIE - International Soc. Optical Engineer, 3513:122-34 (1998)). These micromolding techniques are preferred over other techniques described herein, since they can provide relatively less expensive replication, i.e. lower cost of mass production. In a preferred embodiment, the polymer is biodegradable.

Microneedles, particularly hollow ones and ones formed of relatively brittle materials, may break at the juncture of the microneedle and substrate due to mechanical stresses at the sharp angle formed there. It has been found that the integrity of such microneedles can be improved by reinforcing the base of the microneedles with an additional layer of material (e.g., silicon) applied onto the face of the substrate face adjacent the base end of the microneedles as shown in Figure 17.

### 4. Microneedle Device Applications

The device may be used for single or multiple uses for rapid transport across a biological barrier or may be left in place for longer times (e.g., hours or days) for long-term transport of molecules. Depending on the dimensions of the device, the application site, and the route in which the device is introduced into (or onto) the biological barrier, the device may be used to introduce or remove molecules at specific locations.

As discussed above. Figure 9 shows a side elevational view of a schematic of a preferred embodiment of the microneedle device **10 in** a transdermal application. The device **10** is applied to the skin such that the microneedle **12** penetrate through the stratum corneum and enter the viable epidermis so that the tip of the microneedle at least penetrates into the viable epidermis. In a preferred embodiment, drug molecules in a reservoir within the upper portion **11** flow through or around the microneedles and into the viable epidermis, where the drug molecules then diffuse into the dermis for local treatment or for transport through the body.

To control the transport of material out of or into the device through the microneedles, a variety of forces or mechanisms can be employed. These include pressure gradients, concentration gradients, electricity, ultrasound, receptor binding, heat, chemicals, and chemical reactions. Mechanical or other gates in conjunction with the forces and mechanisms described above can be used to selectively control transport of the material.

In particular embodiments, the device should be "user-friendly." For example, in some transdermal applications, affixing the device to the skin should be relatively simple, and not require special skills. This embodiment of a microneedle may include an array of microneedles attached to a housing containing drug in an internal reservoir, wherein the housing has a bioadhesive coating around the microneedles. The patient can remove a peel-away backing to expose an adhesive coating, and then press the device onto a clean part of the skin, leaving it to administer drug over the course of, for example, several days.

### a. Drug Delivery

Essentially any drug or other bioactive agents can be delivered using these devices. Many drugs can be delivered at a variety of therapeutic rates. The rate can be controlled by varying a number of design factors, including the outer diameter of the microneedle, the number and size of pores or channels in each microneedle, the number of microneedles in an array, the magnitude and frequency of application of the force driving the drug through the microneedle and/or the holes created by the microneedles. For example, devices designed to deliver drug at different rates might have more microneedles for more rapid delivery and fewer microneedles for less rapid delivery. As another example, a device designed to deliver drug at a variable rate could vary the driving force (e.g., pressure gradient controlled by a pump) for transport according to a schedule which was pre-programmed or controlled by, for example, the user or his doctor. The devices can be affixed to the skin or other tissue to deliver drugs continuously or intermittently, for durations ranging from a few seconds to several hours or days.

One of skill in the art can measure the rate of drug delivery for particular microneedle devices using *in vitro* and *in vivo* methods known in the art. For example, to measure the rate of transdermal drug delivery, human cadaver skin mounted on standard diffusion chambers can be used to predict actual rates. *See* Hadgraft & Guy. eds., Transdermal Drug Delivery: Developmental Issues and Research Initiatives (Marcel Dekker, New York 1989); Bronaugh & Maibach. Percutaneous Absorption, Mechanisms--Methodology--Drug Delivery (Marcel Dekker. New York 1989). After filling the compartment on the dermis side of the diffusion chamber with saline, a microneedle array is inserted into the stratum corneum; a drug solution is placed in the reservoir of the microneedle device; and samples of the saline solution are taken over time and assayed to determine the rates of drug transport.

In an alternate embodiment, biodegradable or non-biodegradable microneedles can be used as the entire drug delivery device, where biodegradable microneedles are a preferred embodiment. For example, the microneedles may be formed of a biodegradable polymer containing a dispersion of an active agent for local or systemic delivery. The agent could be released over time, according to a profile determined by the composition and geometry of the microneedles, the concentration of the drug and other factors. In this way, the drug reservoir is within the matrix of one or more of the microneedles.

In another alternate embodiment, these microneedles may be purposefully sheared off from the substrate after penetrating the biological barrier. In this way, a portion of the microneedles would remain within or on the other side of the biological barrier and a portion of the microneedles and their substrate would be removed from the biological barrier. In the case of skin, this can involve inserting an array into the skin, manually or otherwise breaking off the microneedles tips and then removing the base of the microneedles. The portion of the microneedles which remains in the skin or other biological barrier can then release drug over time according to a profile determined by the composition and geometry of the microneedles, the concentration of the drug and other factors. In a preferred embodiment, the microneedles are made of a biodegradable polymer. The release of drug from the biodegradable microneedle tips can be controlled by the rate of polymer degradation. The tips can release drugs for local or systemic effect, or other agents, such as perfume, insect repellent and sun block.

Microneedle shape and content can be designed to control the breakage of microneedles. For example, a notch can be introduced into microneedles either at the time of fabrication or as a subsequent step. In this way, microneedles would preferentially break at the site of the notch. Moreover, the size and shape of the portion of microneedles which break off can be controlled not only for specific drug release patterns, but also for specific interactions with cells in the body. For example, objects of a few microns in size are known to be taken up by macrophages. The portions of microneedles that break off can be controlled to be bigger or smaller than that to prevent uptake by macrophages or can be that size to promote uptake by macrophages, which can be desirable for delivery of vaccines.

### (i). Drugs to be Delivered

Essentially any drug can be delivered using the microneedle devices. As used herein, the term "drug" refers to an agent which possesses therapeutic, prophylactic, or diagnostic properties *in vivo,* for example when administered to an animal, including mammals, such as humans. The drug can be for local treatment or for regional or systemic therapy.

Representative examples of suitable therapeutic and/or prophylactic active agents include proteins, such as hormones, antigens, and growth factors; nucleic acids, such as antisense molecules; and smaller molecules, such as antibiotics, steroids, decongestants, neuroactive agents, anesthetics, and sedatives. Examples of suitable diagnostic agents include radioactive isotopes and radioopaque agents, metals, gases labels including chromatographic, fluorescent or enzymatic labels.

The drug can be or include a peptide, protein, carbohydrate (including monosaccharides, oligosaccharides, and polysaccharides), nucleoprotein, mucoprotein, lipoprotein, glycoprotein, nucleic acid molecules (including any form of DNA such as cDNA, RNA. or a fragment thereof, oligonucleotides, and genes), nucleotide, nucleoside, lipid, biologically active organic or inorganic molecules, or combination thereof.

Drugs can be selected, for example, from enzymes, polysaccharides, polynucleotide molecules, and synthetic organic and inorganic compounds. Representative agents include anti-infectives, hormones, such as insulin, growth regulators, drugs regulating cardiac action or blood flow, and drugs for pain control. The following are representative examples, and disorders they are used to treat. Calcitonin, osteoporosis; Enoxaprin, anticoagulant, Etanercept, rheumatoid arthritis; Erythropoietin, anemia; Fentanyl, postoperative and chronic pain: Filgrastin, low white blood cells from chemotherapy; Heparin, anticoagulant; Insulin, human, diabetes; Interferon β 1a, multiple sclerosis; Lidocaine, local anesthesia; Somatropin, growth hormone; and Sumatriptan, migraine headaches.

The amount of drug can be selected by one of skill in the art based, for example on the particular drug, its desired effect at the planned release levels, and the time span over which the drug should be released.

### (ii). Examples of Drug Delivery Devices

Preferred embodiments of the microneedle device are shown in Figures 20a-c. The device **510** includes substrate **512** from which a three-dimensional array of microneedles **514** protrude. As shown, the annular bore of the microneedles **514** extends through the substrate **512.** The device **510** also includes a reservoir **516** secured to substrate **512** via a sealing mechanism **518.** Figure 20a shows how the reservoir can be accessed directly by application to the skin, for example, for simple transdermal delivery of an agent. The device in Figure 20b includes a deformable bubble reservoir **516.** Manual pressure can be used to expel its contents at the site of application. Figure 20c shows a separate reservoir **516** from means **519** for expelling the contents of the reservoir **516** at the site of administration. The expelling means **519** can be simply a flexible bag. The expelling means **519** may also contain a vacuum so that it expands when vented, to create pressure on the reservoir, or it may be elastic so that it deforms when released from one position (not shown). Alternatively, reservoir **516** could be formed of an elastic material which deforms when released.

The sealing mechanism **518** can be, for example, an adhesive material or gasket. The sealing mechanism **518** can further function as or include a fracturable barrier or rate controlling membrane overlaying the surface of the substrate. In this embodiment, nothing can be released until a seal or peel-off strip covering is removed.

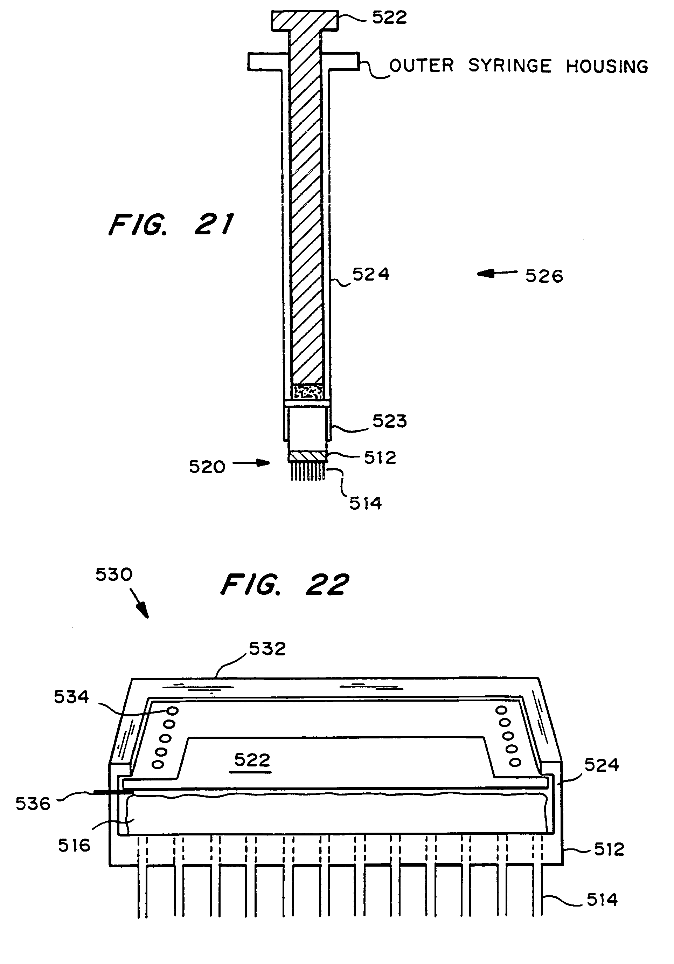

Another preferred embodiment of the microneedle device is shown in Figure 21. The device **520** includes substrate **512** from which a three-dimensional array of microneedles **514** protrude. The device **520** also includes plunger **522** that is slidably secured to the upper surface or substrate 512 by plunger guide frame **524** using a restraint such as a Leur-lock interface **523.** The substrate **512** can be attached or detached to a syringe **526** via a connector such as a Luer-lock type attachment **523.** The plunger **522,** guide frame **524.** and connector **523** connect to, form or contain reservoir **516.** A Luer-lock type attachment could alternatively be used to secure the device to means for controlling flow or transport through the device such as a pump.

A further preferred embodiment of the microneedle device is shown in Figure 22. Like the device in Figure 21, the device **530** includes substrate **512,** microneedles **514,** plunger **522,** plunger guide frame **524,** and reservoir **516.** Device **530** further includes plunger housing **532,** which is attached to, or integrally formed with, plunger guide frame **524.** A compressed spring or other tension-based mechanism **534** is positioned between plunger housing **532** and plunger **522.** The device **530** further includes spring hold/release mechanism **536.** which keeps the plunger up (spring compressed) until triggered to compress reservoir **516.**

### (iii). Initiating Delivery

In a preferred embodiment, delivery of the drug from the reservoir is initiated by applying a force, such as by pressing the top of the reservoir, to cause the reservoir contents (i.e. a drug containing composition) to flow out through the microneedles-an active or dynamic process. For example, the user can apply finger-pressure directly to a deformable reservoir "bubble." (Figure 20) or to a plunger mechanism (Figure 21) or a Luer-lock type syringe that in turn causes the drug composition to be forced from the reservoir. The plunger also can be adapted to activate by application of a constant, reproducible force, for example, a spring (e.g., under compression) (Figure 22) or elastic band (e.g., in tension).

A variation of this embodiment utilizes a balloon-like reservoir in tension to provide the force. Then, when an opening is formed in the balloon reservoir, the contents are forced out of the reservoir as the balloon contracts to its relaxed state. The contraction is selectively triggered to provide the driving force for delivery.

In a preferred embodiment, the force ruptures a fracturable barrier between the reservoir contents and the inlet of the microneedle. Representative barriers include thin foil, polymer, or laminant films. In another preferred embodiment, the microneedles tips are blocked until immediately before use. The blocking material can be, for example, a peelable adhesive or gel film, which will not clog the openings in the microneedle tip when the film is removed from the device.

Delivery also can be initiated by opening a mechanical gate or valve interposed between the reservoir outlet and the microneedle inlet. For example, a thin film or plate can be slid or peeled away from the back of the substrate.

In an alternate embodiment, delivery is initiated by changing the physical or chemical properties of the drug composition and/or of a barrier material. For example, the barrier can be a porous membrane having a porosity that can be selectably altered to permit flow, or the drug composition can be selected to change from a solid or semi-solid state to a fluid state, for example as the temperature is increased from ambient to that of body temperature. Such a drug composition can be prepared, for example, by combining the drug with a biodegradable polymeric material.

A preferred embodiment of the microneedle device is shown in Figure 23a. Figure 23a shows a device **540** in which microneedles **514** attached to a substrate **512** which is attached to multiple compartments **516a, 516b, 516c,** and **516d.** Each compartment can contain or function as a reservoir. Material can be expelled from each compartment through all or a subset of microneedles **514.**

Figure 23b depicts a device **550** in which microneedles **514** are attached to a substrate **512** which is attached to reservoir **558** containing, for example, lyophilized drug **554.** The reservoir **558** is attached to a fracturable barrier **552** which is attached to another reservoir **556** containing, for example, saline. If the barrier **552** is fractured, the two reservoirs **554** and **556** are in fluid communication with each other and their contents can mix.

Delivery also can be initiated by activating an osmotic pump, as described, for example, in U.S. Patent No. 4,320,758 to Eckenhoff, which has been adapted to the substrate of the microneedle device. For example, the reservoir/osmotic pump includes an inner flexible bag that holds the drug charge, an intermediate layer of an osmotically effective solute composition, such as an inorganic salt, that encapsulates the bag, and an outer shape-retaining membrane that is at least in part permeable to water and that encapsulates both the layer of osmotically effective solute composition and the bag. In operation, the bag filled with the fluid drug compositions is exposed to an aqueous environment, so that water is imbibed from the environment by the osmotically effective solute through the membrane into the space between the inner flexible bag and the membrane. Since the bag is flexible and the membrane is rigid, the imbibed water squeezes the bag inwardly, thereby displacing drug out the microneedles.

Figure 24 shows a device **560** in which microneedles **514** are attached to a substrate **512** which is attached to a drug reservoir **562.** This reservoir is surrounded at least partially by a flexible, impermeable membrane **564.** The drug reservoir is connected to another reservoir **566** which contains, for example, an inorganic salt. The two reservoirs **562** and **566** are separated by the impermeable membrane **564.** which is impermeable to the contents of both reservoirs **562** and **566.** The reservoir **566** is also connected to another reservoir **568** which contains, for example, an aqueous solution in which the organic salt is at least partially soluble. The two reservoirs **566** and **568** are separated by a rigid, semi-permeable membrane **570,** which is partially or completely impermeable to the salt in reservoir **566** and partially or fully permeable to the solution in reservoir **568.** There is also an optional fill port or vent **572** in communication with the reservoir **568,** through which material can be added to or removed from the reservoir **568.** Using this device **560.** water can be drawn from the reservoir **568** across the semi-permeable membrane **570** into the reservoir **566** due to osmosis caused by the presence of salt in the reservoir **566.** The flow of water will cause the volume of reservoir **566** to increase and thereby force the volume of reservoir **562** to decrease, which causes material to expel from reservoir **562** through microneedles **514.**

In an alternate embodiment, delivery is initiated by opening the pathway between the reservoir and the microneedle tip, or unblocking the tip openings, and simply allowing the drug to be delivered by diffusion, that is, a passive process.

### (iv). Controlling the Delivery Rate

The microneedle device must be capable of transporting drug across or into the tissue at a useful rate. For example, the microneedle device must be capable of delivering drug at a rate sufficient to be therapeutically useful. The rate of delivery of the drug composition can be controlled by altering one or more of several design variables. For example, the amount of material flowing through the needles can be controlled by manipulating the effective hydrodynamic conductivity (the volumetric through-capacity) of a single device array, for example, by using more or fewer microneedles, by increasing or decreasing the number or diameter of the bores in the microneedles,or by filling at least some of the microneedle bores with a diffusion-limiting material. It is preferred, however, to simplify the manufacturing process by limiting the needle design to two or three "sizes" of microneedle arrays to accommodate, for example small, medium, and large volumetric flows, for which the delivery rate is controlled by other means.

Other means for controlling the rate of delivery include varying the driving force applied to the drug composition in the reservoir. For example, in passive diffusion systems, the concentration of drug in the reservoir can be increased to increase the rate of mass transfer. In active systems, for example, the pressure applied to the reservoir can be varied, such as by varying the spring constant or number of springs or elastic bands.

In either active or passive systems, the barrier material can be selected to provide a particular rate of diffusion for the drug molecules being delivered through the barrier at the needle inlet.

### (v). Feedback about Delivery

In a preferred embodiment, the microneedle device includes a feedback means so that the user can (1) determine whether delivery has been initiated: and/or (2) confirm that the reservoir has been emptied, that is delivery complete. Representative feedback means include a sound, a color (change) indicator, or a change in the shape of a deformable reservoir. In a preferred embodiment, the feedback for completion of delivery is simply that the reservoir is pressed flat against the back of the substrate and cannot be further deformed.

### (vi). Feedback about Penetration of Microneedles into Tissue

The user of the microneedle device typically can determine if the microneedles have been properly inserted into the skin or other tissue through visual or tactile means, that is assessing whether the substrate has been pressed essentially flush to the tissue surface. For example, if a puddle of liquid drug composition appears near the device, then the user may infer that the microneedles are not fully inserted, suggesting that the device needs to be reapplied. The liquid drug compositions can include a coloring agent to enhance the visual feedback.

In a more complex embodiment, an electrical or chemical measurement is adapted to provide the feedback. For example, penetration can be determined by measuring a change in electrical resistance at the skin or other tissue, or a pH change. Alternately, needle-to-needle electrical resistance can be measured. In a preferred embodiment, the microneedle device includes a disposable cartridge containing the microneedles. In these devices, an LED (e.g. green light/red light) or liquid crystal display can be provided with the reusable portion of the device.

### (vii). Multi-Cartridge Microneedle Device

A modification of the disposable, single use microneedle device utilizes a reusable triggering device (e.g., a plunger) in combination with a cartridge containing one or more, preferably a plurality, of single-use microneedle devices. For example, the cartridge can be a circular disk having 10 or 12 microneedle arrays connected to a single-dose reservoir, wherein the cartridge can be loaded into and unloaded from the triggering device. The triggering device can, for example, be designed to move a new dose into position for delivery, compress the reservoir to deliver the drug, and then eject or immobilize the used array. This type of reusable triggering device also can include a power source, such as a battery, used to operate a built-in measurement device, for example, for analyte measurement of interstitial fluids or electrical verification of needle penetration into skin.

### (viii). Microneedle Device Packaging

In a preferred embodiment following manufacture of the microneedle device, it is packaged for storage, shipment, and sale before use. The packaging should prevent contamination and damage. The packaging also should prevent premature triggering or release of any drug or vehicle contents from the reservoir.

It is particularly important that, the microneedle device is provided with a removable protective cover or cushion that protects the microneedles from damage. The protective cover also can function to keep the drug material from prematurely leaking out of the microneedles. In a preferred embodiment, an adhesive material or gel film used to selectively secure the cover over the microneedles. In an alternate embodiment, the film is antiseptic, and following removal can serve as a wipe to prepare the skin surface before insertion of the microneedles.

The packaging also can be adapted to serve as a vessel for safely disposing of the used microneedle device. In a preferred embodiment, the single-use microneedles are provided a device or material to shear the needles from the substrate or plug the microneedles, so as to prevent undesirable reuse of the device. In one embodiment, the inner back of the reservoir is provided with a tacky substance. When the reservoir is compressed against the back of the substrate following delivery of the reservoir contents, the tacky substance is driven into the opening of the microneedles, plugging them. In one embodiment of this device, the tacky material dries and hardens so that the substance cannot readily be removed.

### b. Diagnostic Sensing of Body Fluids Biosensors)

The device is applied to the skin or other biological barrier at the site where the sample is to be collected or measured. Then, biological fluid, or a component thereof, is drawn into or through the pores or bores of the microneedles, and optionally collected in the collection chamber. Alternatively, where the microneedles function as the sensor, sensing occurs without transfer of biological fluid into a collection chamber. One embodiment of the devices described herein may be used to remove material from the body across a biological barrier, i.e. for minimally invasive diagnostic sensing. For example, fluids can be transported from interstitial fluid in a tissue into a reservoir in the upper portion of the device. The fluid can then be assayed while in the reservoir or the fluid can be removed from the reservoir to be assayed, for diagnostic or other purposes. For example, interstitial fluids can be removed from the epidermis across the stratum corneum to assay for glucose concentration, which should be useful in aiding diabetics in determining their required insulin dose.

The sensing device can be in or attached to one or more microneedles, or in a housing adapted to the substrate. Sensing information or signals can be transferred optically (e.g., refractive index) or electrically (e.g., measuring changes in electrical impedance, resistance, current, voltage, or combination thereof). For example, it may be useful to measure a change as a function of change in resistance of tissue to an electrical current or voltage, or a change in response to channel binding or other criteria (such as an optical change) wherein different resistances are calibrated to signal that more or less flow of drug is needed, or that delivery has been completed.

In one embodiment, one or more microneedle devices can be used for (I) withdrawal of interstitial fluid, (2) assay of the fluid, and/or (3) delivery of the appropriate amount of a therapeutic agent based on the results of the assay, either automatically or with human intervention. For example, a sensor delivery system may be combined to from for example, a system which withdraws bodily fluid measures its glucose content, and delivers an appropriate amount of insulin. The sensing or delivery step also can be performed using conventional techniques, which would be integrated into use of the microneedle device. For example, the microneedle device could be used to withdraw and assay glucose, and a conventional syringe and needle used to administer the insulin, or *vice versa.*

In an alternate embodiment, microneedles may be purposefully sheared off from the substrate after penetrating the biological barrier, as described above. The portion of the microneedles which remain within or on the other side of the biological barrier could contain one or more biosensors. For example, the sensor could change color as its output. For microneedles sheared off in the skin, this color change could be observed through the skin by visual inspection or with the aid of an optical apparatus.

The microneedles can also be used for aerosolization or delivery for example directly to a mucosal surface in the nasal or buccal regions or to the pulmonary system.

The microneedle devices disclosed herein also should be useful for controlling transport across tissues other than skin. For example, microneedles can be inserted into the eye across, for example, conjunctiva, sclera, and/or cornea, to facilitate delivery of drugs into the eye. Similarly, microneedles inserted into the eye can facilitate transport of fluid out of the eye, which may be of benefit for treatment of glaucoma. Microneedles may also be inserted into the buccal (oral), nasal, vaginal, or other accessible mucosa to facilitate transport into, out of, or across those tissues. For example, a drug may be delivered across the buccal mucosa for local treatment in the mouth or for systemic uptake and delivery. As another example, microneedle devices may be used internally within the body on, for example, the lining of the gastrointestinal tract to facilitate uptake of orally-ingested drugs or the lining of blood vessels to facilitate penetration of drugs into the vessel wall. For example, cardiovascular applications include using microneedle devices to facilitate vessel distension or immobilization, similarly to a stent, wherein the microneedles/substrate can function as a "staple-like" device to penetrate into different tissue segments and hold their relative positions for a period of time to permit tissue regeneration. This application could be particularly useful with biodegradable devices.These uses may involve invasive procedures to introduce the microneedle devices into the body or could involve swallowing, inhaling, injecting or otherwise introducing the devices in a non-invasive or minimally-invasive manner.

### (i). Biological Fluids for Withdrawal and Sensing

The microneedle devices are useful in the transport of biological fluids from within or across a variety of biological barriers including the skin (or parts thereof): the blood-brain barrier: mucosal tissue: blood vessels: lymphatic vessels: cell membranes: epithelial tissue: and endothelial tissue. The biological barriers can be in humans or other types of animals, as well as in plants, insects, or other organisms, including bacteria, yeast, fungi, and embryos. In preferred embodiments, biological fluids are withdrawn from skin, more preferably human skin, for minimally invasive diagnostic sensing.

Biological fluids useful with the devices described herein include blood, lymph, interstitial fluid, and intracellular fluid. A preferred biological fluid to be withdrawn or sensed is interstitial fluid.

A variety of analytes are routinely measured in the blood, lymph or other body fluids. Examples of typical analytes that can be measured include blood sugar (glucose), cholesterol, bilirubin, creatine, various metabolic enzymes, hemoglobin, heparin, hematocrit, vitamin K or other clotting factors, uric acid, carcinoembryonic antigen or other tumor antigens, and various reproductive hormones such as those associated with ovulation or pregnancy. Other substances or properties desirable to detect include lactate (important for athletes), oxygen, pH, alcohol, tobacco metabolites, and illegal drugs (important for both medical diagnosis and law enforcement).

In a preferred embodiment, interstitial fluids are removed from the dermis or epidermis across the stratum corneum to assay for glucose concentration, which is useful in aiding diabetics in determining their required insulin dose.

### (ii). Feedback about Microneedle Penetration into Tissue

The user of the microneedle device typically can determine if the microneedles have been properly inserted into the skin or other tissue through visual or tactile means, that is assessing whether the substrate has been pressed essentially flush to the tissue surface. A coloring agent can be used to enhance the visual feedback by indicating a color change triggered by biological fluid filing the collection chamber.

In a more complex embodiment, an electrical or chemical measurement is adapted to provide the feedback. For example, penetration can be determined by measuring a change in electrical resistance at the skin or other tissue, or a pH change. Alternately, needle-to-needle electrical resistance can be measured. In a preferred embodiment, the microneedle device includes a disposable cartridge containing the microneedles. In these devices, an LED (e.g., green light/red light) or liquid crystal display can be provided with the reusable portion or the device.

### (iii). Feedback about Withdrawal

In a preferred embodiment, the microneedle device includes a feedback means so that the user can determine whether withdrawal and/or sensing has been completed. Representative feedback means include a sound, a color (change) indicator, or a change in the position or shape of a reservoir or collection chamber.

### (iv). Initiating and Controlling Fluid Withdrawal

In a preferred embodiment, the microneedle device is capable of transporting molecules across or from the tissue at a useful rate. That rate will, of course, depend on the particular application. For assaying blood glucose, for example, the device preferably withdraws at least between about 5 and 10 µl of interstitial fluid, preferably in about a minute or less time. For other applications, it may be desirable to apply the device to the barrier for several hours or more in order to monitor the changing blood plasma level of a particular analyte, such as an exogenous drug or hormone level, for example on a real-time basis or to obtain a time-averaged value.

The rate of withdrawal can be controlled by altering one or more of several design variables. For example, the amount of material flowing through the needles can be controlled by manipulating the volumetric through-capacity of a single device array, e.g., by using more or fewer microneedles, by increasing or decreasing the number or diameter of the bores in the microneedles, by filling at least some of the microneedle bores with a diffusion-limiting material, or by varying the driving force applied to the biological fluid. It is preferred, however, to simplify the manufacturing process by limiting the needle design to two or three "sizes" of microneedle arrays to accommodate, for example small, medium, and large volumetric flows, for which the withdrawal rate is controlled by other means.

The flow of biological fluid from the tissue through the microneedle and into the collection chamber or into contact with the sensors occurs due to capillary action, diffusion, or is induced using conventional mechanical pumps or nonmechanical driving forces, such as electroosmosis or electrophoresis, thermal gradients, or convection. For example, in electroosmosis, electrodes are positioned on the biological barrier surface, on one or more microneedles, and/or on the substrate adjacent the needles, to create a convective flow which carries charged ionic species and/or neutral molecules toward and/or through at least one of the microneedles.

Transportation of molecules through the microneedles can be controlled or monitored using, for example, various combinations of semi-permeable membranes, valves, pumps, sensors, actuators, and microprocessors. These components can be produced using standard manufacturing or microfabrication techniques. Actuators that may be useful with the microneedle devices disclosed herein include micropumps, microvalves, and positioners.

In preferred embodiments, the microneedle device is used with another mechanism that enhances the permeability of the biological barrier, such as by increasing cell membrane disruption, using electric fields, ultrasound, chemical enhancers, vacuum, viruses, pH, heat, and/or light. Chemical and/or physical enhancers can be applied to the biological barrier at the insertion site of the device before or during the withdrawal of the device, for example, to draw analyte closer to the skin surface.

### (a). bulk flow extraction

A preferred microneedle device is shown in Figure 26 and described above. Microneedle device **620** preferably is operated as follows. After or simultaneously with the insertion of device **620** into, for example, the skin, manual pressure is applied to elastic cap **622** so as to deform (compress) it and reduce the interior volume of elastic cap **622** and fluid collection chamber **616.** The deformation causes gas within the cap and chamber to be purged from the device via one-way valve **624**. As elastic cap **622** returns to its undeformed (uncompressed) shape, pressure is reduced within the interior volume, creating a pressure differential or vacuum, which causes the interstitial fluid, to be drawn through the microneedles **614** and into fluid collection chamber **616,** until the pressure is equalized. Once in the collection chamber **616,** the fluid sample can be analyzed by withdrawing device **620** from the skin, again depressing elastic cap **622,** to force the sample out of the chamber either through the microneedles **614** or one-way valve **624,** and, for example, onto a diagnostic strip or into a container adapted for the particular analytical method to be employed.

Other spring means can be adapted to cause a change in the volume, and thus internal pressure, of the collection chamber. For example, the fluid collection chamber can be adapted to have a movable, rigid top with fixed, rigid side walls, wherein the interface between the walls and top form a gas-tight seal (such as by using a gasket). The volume of the piston-like apparatus can be expanding by activating one or more compressed springs to move the top of the chamber as desired, thereby reducing the pressure in the collection chamber.

In an alternative embodiment, withdrawal can be initiated by activating an osmotic pump, as described, for example, in U.S. Patent No. 4,320,758 to Eckenhoff. which has been engineered to create a volume expansion/pressure reduction in the collection chamber to draw biological fluid to be drawn into the collection chamber.

In a preferred embodiment, withdrawal is conducted using a microneedle device fitted to a Luer-Lock syringe or similar conventionally-used devices that currently uses hypodermic needles in the barrier penetration method.

### (b) diffusion-based extraction

One preferred embodiment uses diffusion to move the analyte fluid into the fluid collection chamber. A microneedle device using this method generally includes microneedle bores or pores, and/or a fluid collection chamber, that are filled with a diffusion medium, such as water, a saline solution, or a gel, which is physiologically compatible with the biological barrier tissue. Once the needle is inserted into the biological barrier (e.g., the skin), the diffusion medium contacts the biological fluid. Typically, the diffusion medium in the microneedles initially contains none of the analyte to be measured: therefore, analyte at a higher concentration in the biological fluid diffuses into the diffusion medium in the microneedle until a detectable level of analyte in collected or measured. This should be achieved rapidly due to the very small size/length of the microneedles, thereby providing an analyte concentration for sensing that is rapidly response to that in the tissue.

### (c), hybrid bulk flow/diffusion

In another embodiment, the microneedle device is designed to employ a combination of forces to drive the analyte into the collection chamber. A preferred hybrid design includes the preferred bulk flow microneedle device described above, but with two primary differences. First, the fluid collection chamber has no one-way valve and contains a fluid diffusion medium, preferably physiological saline. Second, a temporary barrier is interposed between the fluid collection chamber and the base of the microneedles. The temporary barrier can be a fracturable membrane, mechanical gate, or other means for containing the diffusion medium within the device until the barrier is broken or removed at a selected time to permit flow of molecules between the microneedles and fluid collection chamber.

In a preferred embodiment, the temporary barrier is an easily fracturable membrane. The device can function as follows: The microneedles first are inserted into the skin of the user. Then the user depresses the elastic cap, causing the fluid diffusion medium to fracture the membrane and fill the bores or pores of the microneedles and contact the biological fluid. After the user releases the elastic cap, it partially or fully returns to its undeformed shape, reducing the pressure in the interior and causing a mixture of the biological fluid and diffusion medium to be drawn through the microneedles and into fluid collection chamber. Once bulk fluid flow ceases, diffusion may continue until the concentration of biological fluid or analyte equilibrates. The mixture can be assayed as described herein, considering the dilution effect induced by the presence of the diffusion medium.

Any of these embodiments of the microneedle device can be adapted to function a series of micro-assays (e.g., as can be provided with an electronics package) or single assays (e.g., standard glucose strips).

### (v). Multi-Cartridge Microneedle Device

A modification of the disposable, single use microneedle device utilizes a reusable portion providing the withdrawal force (e.g., suction) or electronics package in combination with a cartridge containing one or more, preferably a plurality, of single-use, disposable microneedle devices for collection and/or sensing. For example, the cartridge can be a circular disk having 10 or 12 microneedle arrays connected to a single-assay element, wherein the cartridge can be loaded into and unloaded from the reusable portion. The triggering device can, for example, be designed to move a new microneedle array into position, withdraw or sense the sample of biological fluid, and then eject or immobilize the used microneedle array. This type of reusable triggering device also can include a power source, such as a battery, used to operate a built-in measurement device, for example, for analyte measurement of interstitial fluids. In one embodiment, the devices are provided in a multipack (e.g., six or twelve pack) form of complete devices, that is, each device is fully sufficient for use. Individual devices can be separated as needed for each sensing measurement.

### c. Delivery of Energy

Other than transport of drugs and biological molecules, the microneedles may be used to transmit or transfer other materials and energy forms, such as light, electricity, heat, or pressure. The microneedles, for example, could be used to direct light to specific locations within the body, in order that the light can directly act on a tissue or on an intermediary, such as light-sensitive molecules in photodynamic therapy.

The present invention will be further understood with reference to the following non-limiting examples.

### Example 1: Fabrication of Solid Silicon Microneedles

A chromium masking material was deposited onto silicon wafers and patterned into dots having a diameter approximately equal to the base of the desired microneedles. The wafers were then loaded into a reactive ion etcher and subjected to a carefully controlled plasma based on fluorine/oxygen chemistries to etch very deep, high aspect ratio valleys into the silicon. Those regions protected by the metal mask remain and form the microneedles.

<100>-oriented, prime grade, 450-550 µm thick, 10-15 Ω-cm silicon wafers (Nova Electronic Materials Inc.. Richardson. TX) were used as the starting material. The wafers were cleaned in a solution of 5 parts by volume deionized water, 1 part 30% hydrogen peroxide, and 1 part 30% ammonium hydroxide (J.T. Baker, Phillipsburg, NJ) at approximately 80°C for 15 minutes, and then dried in an oven (Blue M Electric, Watertown, WI) at 150°C for 10 minutes. Approximately 1000 Å of chromium (Mat-Vac Technology, Flagler Beach, FL) was deposited onto the wafers using a DC-sputterer (601 Sputtering System, CVC Products, Rochester. NY). The chromium layer was patterned into 20 by 20 arrays of 80 µm diameter dots with 150 µm center-to-center spacing using the lithographic process described below.

A layer of photosensitive material (1827 photoresist. Shipley, Marlborough, MA) was deposited onto the chromium layer covering the silicon wafers. A standard lithographic mask (Telic. Santa Monica. CA) bearing the appropriate dot array pattern was positioned on top of the photoresist layer. The wafer and photoresist were then exposed to ultraviolet (UV) light through the mask by means of an optical mask aligner (Hybralign Series 500. Optical Associates, Inc., Milpitas, CA). The exposed photoresist was removed by soaking the wafers in a liquid developer (354 developer. Shipley, Marlborough, MA) leaving the desired dot array of photoresist on the chromium layer. Subsequently, the wafers were dipped into a chromium etchant (CR-75; Cyanteck Fremont. CA), which etched the chromium that had been exposed during the photolithography step, leaving dot arrays of chromium (covered with photoresist) on the surface of the silicon wafer. The photoresist still present on the chromium dots formed the masks needed for fabrication of the microneedles, described below.

The microneedles were fabricated using a reactive ion etching techniques based on the Black Silicon Method developed at the University of Twente. The patterned wafers were etched in a reactive ion etcher (700 series wafer/batch Plasma Processing System. Plasma Therm, St. Petersburg, FL) with means for ensuring good thermal contact between the wafers and the underlying platen (Apiezon N, K.J. Lesker. Clairton. PA). The wafers were etched using the following gases and conditions: SF₆ (20 standard cubic centimeters per minute) and O₂ (15 standard cubic centimeters per minute) at a pressure of 150 mTorr and a power of 150 W for a run time of approximately 250 minutes. These conditions caused both deep vertical etching and slight lateral underetching. By controlling the ratio of flow rates of the SF₆ and O₂ gases used to form the plasma, the aspect ratio of the microneedles could be adjusted. The regions protected by the chromium masks remained and formed the microneedles. Etching was allowed to proceed until the masks fell off due to underetching, resulting in an array of sharp silicon spikes.

### Example 2: Transdermal Transport Using Solid Microneedles

To determine if micro fabricated microneedles could be used to enhance transdermal drug delivery, arrays of microneedles were made using a deep plasma etching technique. Their ability to penetrate human skin without breaking was tested and the resulting changes in transdermal transport were measured.

Arrays of microneedles were fabricated having extremely sharp tips (radius of curvature less than 1 µm), and are approximately 150 µm long. Because the skin surface is not flat due to dermatoglyphics and hair, the full length of these microneedles will not penetrate the skin. All experiments were performed at room temperature (23±2 °C).

The ability of the microneedles to pierce skin without breaking was then tested. Insertion of the arrays into skin required only gentle pushing. Inspection by light and electron microscopy showed that more than 95% of microneedles within an array pierced across the stratum corneum of the epidermis samples. Moreover, essentially all of the microneedles that penetrated the epidermis remained intact. On those very few which broke, only the top 5-10 µm was damaged. Microneedle arrays could also be removed without difficulty or additional damage, as well as reinserted into skin multiple times.

To quantitatively assess the ability of microneedles to increase transdermal transport, calcein permeability of human epidermis with and without inserted microneedle arrays was measured. Calcein crosses skin very poorly under normal circumstances and therefore represents an especially difficult compound to deliver. As expected, passive permeability of calcein across unaltered skin was very low, indicating that the epidermis samples were intact.

Insertion of microneedles into skin was capable of dramatically increasing permeability to calcein. When microneedles were inserted and left embedded in the skin, calcein permeability was increased by more than 1000-fold. Insertion of microneedles for 10 s, followed by their removal, yielded an almost 10,000-fold increase. Finally, insertion of a microneedle array for 1 h. followed by its removal, increased skin permeability by about 25,000-fold. Permeabilities for skin with microneedles inserted and then removed are higher than for skin with microneedles remaining embedded probably because the microneedles themselves or the silicon plate supporting the array may block access to the microscopic holes created in the skin. Light microscopy showed that the holes which remained in the skin after microneedles were removed were approximately 1 µm in size.

To confirm *in vitro* experiments which showed that skin permeability can be significantly increased by microneedles, studies were conducted with human volunteers. They indicated that microneedles could be easily inserted into the skin of the forearm or hand. Moreover, insertion of microneedle arrays was never reported to be painful, but sometimes elicited a mild "wearing" sensation described as a weak pressure or the feeling of a piece of tape affixed to the skin. Although transport experiments were not performed *in vivo,* skin electrical resistance was measured before and after microneedle insertion. Microneedles caused a 50-fold drop in skin resistance, a drop similar to that caused by the insertion of a 30-gauge "macroneedle." Inspection of the site immediately after microneedle insertion showed no holes visible by light microscopy. No erythema, edema, or other reaction to microneedles was observed over the hours and days which followed. This indicates that microneedle arrays can permeabilize skin in human subjects in a non-painful and safe manner.

### Example 3: Fabrication of Silicon Microtubes

Three-dimensional arrays of microtubes were fabricated from silicon, using deep reactive ion etching combined with a modified black silicon process in a conventional reactive ion etcher. The fabrication process is illustrated in Figures 4a-d. First, arrays of 40 µm diameter circular holes **32** were patterned through photoresist **34** into a 1 µm thick SiO₂ layer **36** on a two inch silicon wafer **38** (Figure 4a). The wafer **38** was then etched using deep reactive ion etching (DRIE) (Laermer, et al., "Bosch Deep Silicon Etching: Improving Uniformity and Etch Rate for Advanced MEMS Applications," Micro Electro Mechanical Systems, Orlando, Florida. USA (Jan. 17-21, 1999)), in an inductively coupled plasma (ICP) reactor to etch deep vertical holes **40.** The deep silicon etch was stopped after the holes 40 are approximately 200 µm deep into the silicon substrate **38** (Figure 4b) and the photoresist **34** was removed. A second photolithography step patterned the remaining SiO₂ layer **36** into circles concentric to the holes, thus leaving ring shaped oxide masks **34** surrounding the holes (Figure 4c). The photoresist **34** was then removed and the wafer **38** was again deep silicon etched, while simultaneously the holes **40** were etched completely through the wafer **38** (inside the SiO₂ ring) and the silicon was etched around the SiO₂ ring **38** leaving a cylinder **42** (Figure 4d). The resulting tubes were 150 µm in height, with an outer diameter of 80 µm, an inner diameter of 40 µm, and a tube center-to-center spacing of 300 µm.

### Example 4: Micromold Fabrication of Metal Microtubes

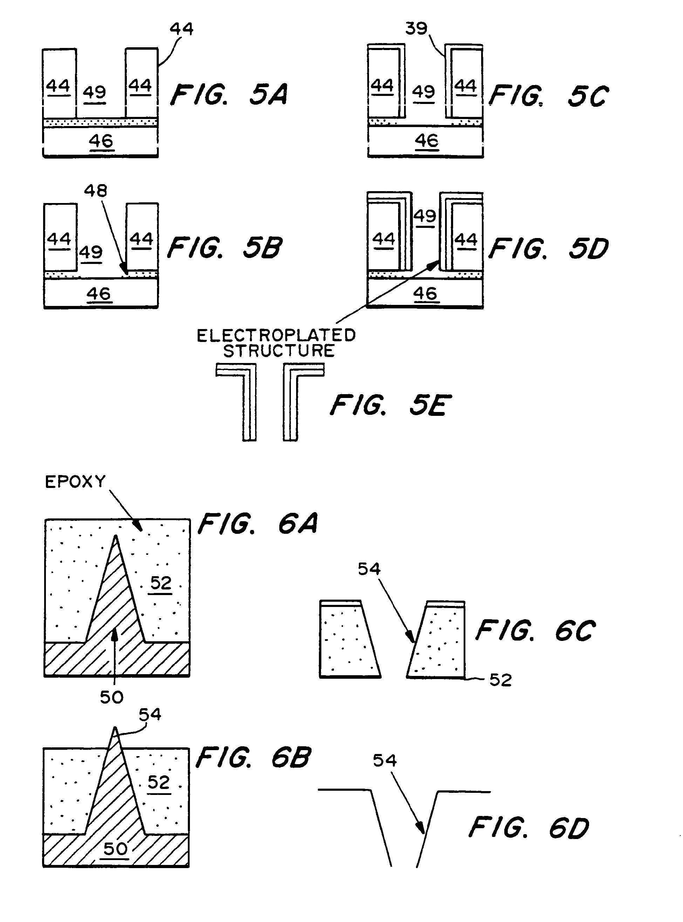

Hollow metal microtubes were prepared without dry silicon etching, using a thick, photo-defined mold of epoxy. The sequences are illustrated in Figures 5a-e. First, a thick layer of SU-8 epoxy **44** was spin cast onto a silicon or glass substrate **46** that had been coated with 30 nm of titanium **48.** the sacrificial layer. Arrays of cylindrical holes **49** were then photolithographically defined through an epoxy layer **44,** typically 150 µm thick (Figure 5a). The sacrificial layer then was partially removed, using a wet etching solution containing hydrofluoric acid and water at the bottom of the cylindrical holes in the SU-8 photoresist **46** (Figure 5b). A seed layer of Ti/Cu/Ti (30 nm/200 nm/30 nm) **39** was then conformally DC sputter-deposited onto the upper surface of the epoxy mold and onto the sidewalls of the cylindrical holes **49** (Figure 5c). As shown in Figure 5c, the seed layer **39** was electrically isolated from the substrate. Subsequently. NiFe was electroplated onto the seed layer 39 (Figure 5d), the epoxy **44** was removed from the substrate, and the surrounding epoxy **44** was removed (Figure 5e). The resulting microtubes are 200 µm in height with an outer diameter of 80 µm, an inner diameter of 60 µm, and a tube center-to-center spacing of 150 µm. The holes in the interior of the microtubes protrude through the base metal supporting the tubes.

### Example 5: Micromold Fabrication of Tapered Microneedles

A micromold having tapered walls was fabricated by molding a preexisting 3-D array of microneedles, i.e. the mold-insert, and subsequently removing the mold insert. The micromold was then surface plated in a manner similar to that for the microtubes described in Example 4. The fabrication sequence is illustrated in Figures 6a-d.

First, an array of solid silicon microneedles **50** were prepared as described in Henry, et al., "Micromachined Needles for the Transdermal Delivery of Drugs," Micro Electro Mechanical Systems, Heidelberg, Germany, Jan. 26-29, pp. 494-98 (1998). Then, a layer of epoxy **52** (SU**-**8) was spin cast onto the microneedle array to completely blanket the array (Figure 6a). The epoxy **52** settled during pro bake to create a planar surface above the tips of the microneedles **50.** The epoxy **52** was then fully pre-baked, photolithographically cross-linked, and post-baked.

Then, the upper surface of the epoxy **52** was etched away using an O₂/CHF₃ plasma until approximately 1 to 2 µm of the needle tips **54** were exposed, protruding from the epoxy **52** (Figure 6b). The silicon was then selectively removed by using a SF₆ plasma (Figure 6c). The remaining epoxy mold **52** provided a negative of the microneedles with a small diameter hole where the tip of the silicon needle protruded. After the removal of the silicon, a seed layer of Ti-Cu-Ti **54** was conformally sputter-deposited onto the top and sidewalls of the epoxy micromold **52.** Following the same process sequence as described in Example 4. NiFe was then electroplated onto the seed layer **54** (Figure 6c). Finally, the epoxy was removed using an O₂/CHF₃ plasma, leaving a 20 x 20 array of NiFe hollow metal microneedles **54** (Figure 6d). The microneedles **54** were 150 µm in height with a base diameter of 80 µm, a tip diameter of 10 µm, and a needle-to-needle spacing of 150 µm.

Micromold-based microneedles also have been successfully manufactured using a process in which the epoxy mold material was replaced with PDMS. In this case, it was possible to remove the mold from the mold insert, as well as the microneedles from the mold, using only physical techniques such as peeling. This approach advantageously requires no dry etching and allows one to reuse both the mold and the mold insert.

### Example 6: Micromold Fabrication of Tapered Microneedles Using Laser-Formed Molds

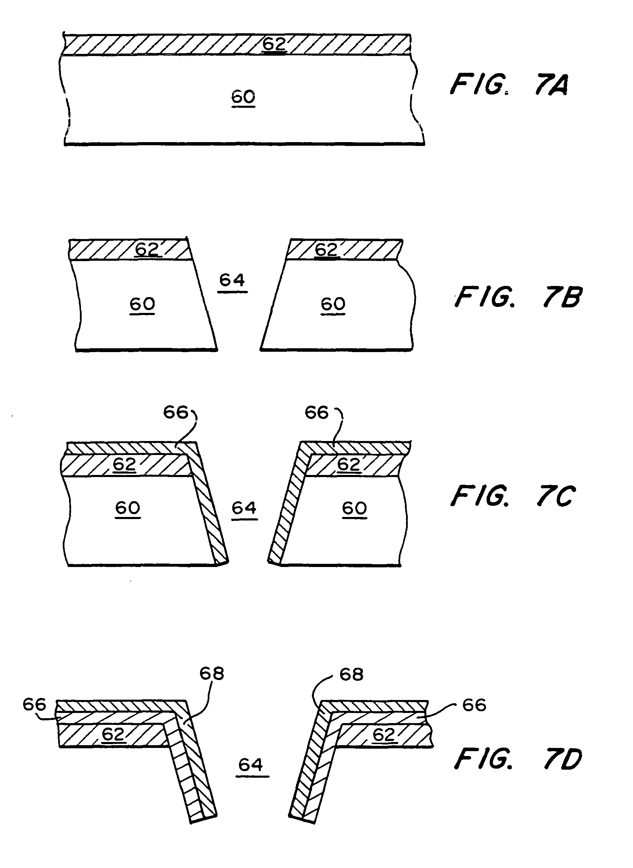

A micromold having tapered walls was fabricated by use of laser ablation techniques, as shown in Figures 7a-d. A laser-ablatable polymer sheet 60 such as KAPTON^{™} polyimide approximately 150 µm in thickness was optionally laminated to a thin (10-30 µm) metal sheet **62** such as titanium (Figure 7a). A tapered hole **64** was formed in the metal/polymer laminate **60/62** using a laser technique such as excimer laser ablation (Figure 7b). The entry hole of the laser spot was on the metal side **62,** and a through hole was made through both the metal sheet and the polymer film. The through hole **64** was tapered in combination with either defocusing or appropriate substrate motion to create a taper such that the wide end of the hole **64** (typically 40-50 µm) was on the metal side **62** and the narrow end of the hole **64** (typically 10-20, µm) was on me polymer **60** side, 4 thin layer of metal **66.** e.g. titanium, of thickness 0.1 µm was then deposited. e.g., using a sputter-deposition technique, in such a way that the metal **66** deposited on the metal film side and coated the polymer sidewalls, but did not coat the polymer 60 side of the laminate (Figure 7c). Electrodeposition of metal **68,** e.g., gold, to a thickness of 1 to 5 µm was then performed on the titanium-coated metal surface **66.** and polymer sidewalls curved section of **60** next to **64.** Finally, the polymer **60** was removed, using e.g. an oxygen plasma, to form the completed microneedles (Figure 7d).

Alternate polymer removal methods, such as thermal, solvent, aqueous, or photo-degradation followed by solvent or aqueous removal, are also possible if the polymer material is chosen appropriately (e.g., a photoresist resin).

### Example 7: Formation of Microneedles by Embossing

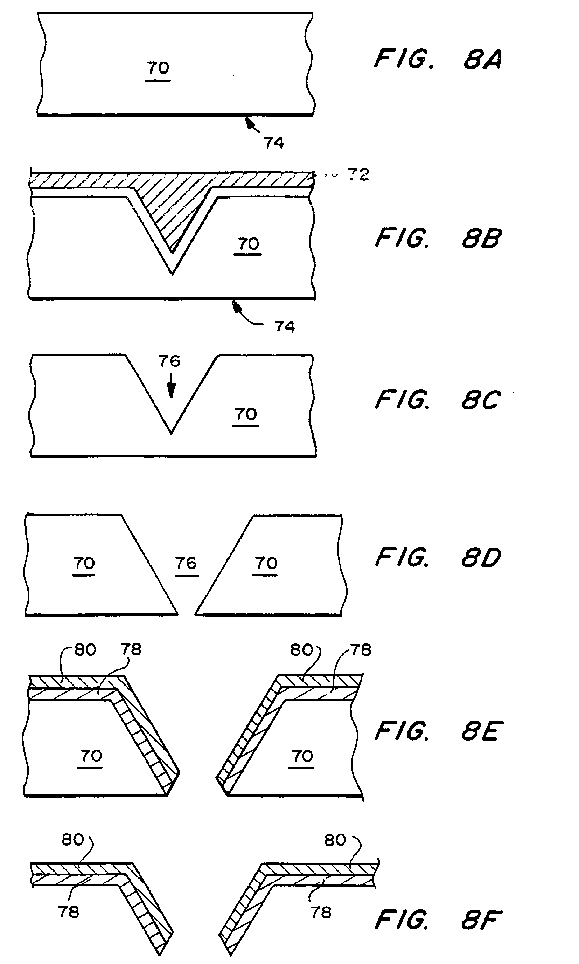

Formation of a microneedle by embossing is shown in Figures **8a****-f.** A polymeric layer **70** (Figure 8a) is embossed by a solid microneedle or microneedle array **72** (Figure 8b). The array **72** is removed (Figure 8c), and the layer **70** is etched from the non-embossed side **74** until the embossed cavity 76 is exposed (Figure 8d). A metallic layer **78** is then deposited on the embossed side and the sidewalls, but not on the non-embossed side **74** (Figure 8e). This layer **78** is optionally thickened by electrodeposition of an additional metal layer **80** on top of it (Figure 8e). The polymer layer **70** is then removed to form the microneedles **78/80** (Figure 8f).

### Example 8: Transdermal Application of Hollow Microneedles

The bore of hollow microneedles must provide fluid flow with minimal clogging in order to be suitable to transport material, such as in transdermal drug delivery. Therefore, microneedles and microtubes were evaluated to determine their suitability for these functions.

Hollow metal and silicon microneedles, produced as described in Examples 3-5. were inserted through human skin epidermis with no apparent clogging of the needle bores. Scanning electron microscopy of a hollow metal (NiFe) microneedle penetrating up through the underside of human epidermis showed the microneedle remains intact, with the tip free of debris. Similarly, silicon microneedles, metal microneedles, and metal microtubes were successfully inserted through human skin. Also, the hollow microneedles were shown to permit the flow of water through their bores.

### Example 9: Drug Transport Through Microneedles Inserted Into Skin

Studies were performed with solid and hollow microneedles to demonstrate transport of molecules and fluids. As shown in Table 1. transport of a number of different compounds across skin is possible using microneedles. These studies were performed using either solid silicon microneedles or using hollow silicon microneedles made by methods described in this patent. Transport was measured across human cadaver epidermis *in vitro* using Franz diffusion chambers at 37 °C using methods described in Henry, et al., "Microfabricated microneedles: A novel method to increase transdermal drug delivery'' J. Pharm. Sci. 87: 922-25 (1998).

The transdermal delivery of calcein, insulin, bovine serum albumin ("BSA"), and nanoparticles was measured. Delivery refers to the ability to transport these compounds from the stratum corneum side of the epidermis to the viable epidermis side. This is the direction of transport associated with delivering drugs into the body. Removal of calcein was also measured. Removal refers to the ability to transport calcein from the viable epidermis side of the epidermis to the stratum corneum side. This is the direction of transport associated with removing from the body compounds found in the body, such as glucose.

In all cases shown in Table 1, transport of these compounds across skin occurred at levels below the detection limit when no needles were inserted into the skin. Intact skin provides an excellent barrier to transport of these compounds. In all cases examined, when solid microneedles were inserted into the skin and left in place, large skin permeabilities were measured, indicating that the microneedles had created pathways for transport across the skin. Furthermore, in all cases, when solid microneedles were inserted into the skin and then removed, even greater skin permeabilities resulted. Finally, when hollow microneedles were inserted into the skin and left in place, still greater skin permeabilities resulted for those compounds tested. These studies show that micro needles can dramatically increase skin permeability and can thereby increase transport of a number of different compounds across the skin. They also shows that when solid microneedles are used, a preferred embodiment involves inserting and then removing microneedles, rather than leaving them in place. They also shows that using hollow microneedles are a preferred embodiment over the use of solid microneedles.

**Table 1: Transport of Drugs Through Microneedles Inserted Into Skin**

| Compound | No needles | Solid needles inserted | Solid needles inserted and removed | Hollow needles inserted |
|---|---|---|---|---|
| Calcein delivery | ** | 4 x 10⁻⁵ | 1 x 10⁻² | 1 x 10⁻¹ |
| Calcein removal | ** | 2 x 10⁻³ | 1 x 10⁻² | n.a. |
| Insulin delivery | ** | 1 x 10⁻⁴ | 1 x 10⁻² | n.a. |
| BSA delivery | ** | 9 x 10⁻⁴ | 8 x 10⁻³ | 9 x 10⁻² |
| Nanoparticle delivery | ** | n.a. | 3 x 10⁻⁵ | n.a. |

| | | | | |
|---|---|---|---|---|
| ** means that the transport was below the detection limit. n.a. means that the data are not available. Nanoparticles were made of latex with a diameter of approximately 100 nm. | | | | |

### Example 10: Flow of Water Through Hollow Microneedles

To demonstrate that fluid can be forced through hollow microneedles at meaningful rates, the flow rate of water through a microneedle array was measures as a function of pressure. The array used contained 100 hollow silicon microneedles having an inner diameter of 50 µm and an outer diameter of 80 µm. The results, which are shown in Table 2. demonstrate that significant flow rates of water through microneedles can be achieved at modest pressures. The measured flow rates are comparable to flow rates through hypodermic needles attached to syringes.

**Table 2: Flow Rate of Water Through Hollow Silicon Microneedles Function of Applied Pressure**

| Pressure (Psi/kPa) | Flow rate (ml/min) |
|---|---|
| 1.0/6,89 | 16 |
| 1.5/10,33 | 24 |
| 2.0/13,78 | 31 |
| 2.5/17,22 | 38 |
| 3.0/20,67 | 45 |

Modifications and variations of the methods and devices described herein will be obvious to those skilled in the art from the foregoing detailed description. Such modifications and variations are intended to come within the scope of the appended claims.

## Claims

1. A device 120, 130, 150, 160, 176 for transport of a material or energy across or into a deformable elastic biological barrier comprising
a) a plurality of hollow microneedles 124, 140, 154, 162, 174 each having a base end and a tip, with at least one hollow pathway disposed at or between the base end and the tip,
b) a substrate (122) to which the base ends of the microneedles 124, 140, 154, 162, 174 are attached or integrated, and
c) at least one reservoir (126, 136) which is in connection with the base ends of at least one of the microneedles, either integrally or separably until the moment of use,
wherein the volume or amount of material to be transported can selectively be altered, **characterised in that** the device 120, 130, 150, 160, 176 comprises means (128, 132, 152, 164, 170) for improving penetration of the barrier by the microneedles 124, 140, 154, 162, 174 by limiting the elasticity of the barrier.

2. The device 120, 130, 150, 160, 176 of claim 1 wherein the reservoir (126, 136) is formed of a material which is deformable.

3. The device 120, 130, 150, 160, 176 of claim 2 wherein the reservoir (126, 136) is elastic.

4. The device 120, 130, 150, 160, 176 of claim 1 wherein the reservoir (126, 136) further comprises a therapeutic, prophylactic, or diagnostic agent.

5. The device 120, 130, 150, 160, 176 of claim 4 wherein the agent is selected from the group consisting of peptides, proteins, carbohydrates, nucleic acid molecules, lipids, organic molecules, biologically active inorganic molecules, and combinations thereof.

6. The device 120, 130, 150, 160, 176 of claim 1 further comprising a plunger (522) movably secured to the substrate, wherein the plunger (522) can compress the reservoir (126, 136),

7. The device 120, 130, 150, 160, 176 of claim 6 further comprising a spring (534) engaged with the pluniger (522).

8. The device 120, 130, 150, 160, 176 of claim 1 wherein the diameter of the microneedles 124, 140, 154, 162, 174 is between 10 µm and 200 µm.

9. The device 120, 130, 150, 160, 176 of claim 1 wherein the length of the microneedles 124, 140, 154, 162, 174 is between 100 µm and 1 mm.

10. The device 120, 130, 150, 160, 176 of claim 1 wherein the microneedles 124, 140, 154, 162, 174 are adapted to provide an insertion depth of less than 100 to 150 µm.

11. The device 120, 130, 150, 160, 176 of claim 1 comprising; a three dimensional array of microneedles 124, 140, 154, 162, 174.

12. The device 120, 130, 150, 160, 176 of claim 1 further comprising an adhesive material for securing the device 120, 130, 150, 160, 176 during delivery.

13. The device 120, 130, 150, 160, 176 of claim 1 wherein the reservoir (126, 136) is a syringe or pump connected to the substrate.

14. The device 120, 130, 150, 160, 176 of claim 1 comprising an attachment means for securing the device to a syringe or pump.

15. The device 120, 130, 150, 160, 176 of claim 13 wherein the substrate is adapted to removably connect to a standard or Luer-lock syringe.

16. The device 120, 130, 150, 160, 176 of claim 1 further comprising a sealing mechanism (518) which is interposed between the reservoir (126, 136) and the substrate (122) or which is secured to the tips of the microneedles 124, 140, 154, 162, 174.

17. The device 120, 130, 150, 160, 176 of claim 16 wherein the sealing mechanism (518) is a fracturable barrier interposed between the reservoir (126, 136) and the substrate (122).

18. The device 120, 130, 150, 160, 176 of claim 1 further comprising a means for providing feedback to indicate that delivery has been initiated or completed.

19. The device 120, 130, 150, 160, 176 of claim 1 wherein at least the microneedles 124, 140, 154, 162, 174 are manufactured for single use as a disposable reagent.

20. The device 120, 130, 150, 160, 176 of claim 1 wherein the device 120, 130, 150, 160, 176 further comprises means for stopping delivery.

21. The device 120, 130, 150, 160, 176 of claim 1 further comprising an osmotic pump to move agent.

22. The device 120, 130, 150, 160, 176 of claim 1 wherein the microneedle 124, 140, 154, 162, 174 is at an angle other than perpendicular to the substrate (122).

23. The device 120, 130, 150, 160, 176 of claim 1 wherein the substrate (122) is formed of a flexible material.

24. The device 120, 130, 150, 160, 176 of claim 1 comprising multiple compartments or reservoirs (126, 136)

25. The device 120, 130, 150, 160, 176 of claim 24 wherein the compartments or reservoirs (126, 136) can be connected or in communication with each other.

26. The device 120, 130, 150, 160, 176 of claim 24 wherein one or more reservoirs (126, 136) contain a solid formulation and one or more reservoirs (126, 136) contains a liquid carrier for the solid formulation.

27. The device 120, 130, 150, 160, 176 of claim 1 in a packaging that protects the device 120, 130, 150, 160, 176 from damage and/or contamination.

28. The device 120, 130, 150, 160, 176 of claim 1 further comprising a removable liner that covers the microneedle tips and that can be used to protect and/or seal access to or from the microneedles 124, 140, 154, 162, 174.

29. The device 120, 130, 150, 160, 176 of claim 1 wherein the device 120, 130, 150, 160, 176 comprises rate control means, which can be used to regulate the rate or extent at which materials flow through the microneedles 124, 140, 154, 162, 174.

30. The device 120, 130, 150, 160, 176 of claim 29 wherein the rate control means is a membrane at one end of the microneedles 124, 140, 154, 162, 174 through which agent or fluid must pass.

31. The device 120, 130, 150, 160, 176 of claim 29 wherein the rate control means is a material positioned within the hollow pathway of the microneedles 124, 140, 154, 162, 174.

32. The device 120, 130, 150, 160, 176 of claim 1 comprising multiple microneedle arrays in combination with means for accessing one or more arrays at a time.

33. The device 120, 130, 150, 160, 176 of claim 1 comprising means for preventing undesired contact with or use of the microneedles 124, 140, 154, 162, 174.

34. The device 120, 130, 150, 160, 176 of claim 33 wherein the means is packaging that covers the microneedles 124, 140, 154, 162, 174.

35. The device 120, 130, 150, 160, 176 of claim 33 wherein the means is packaging that shears off the microneedles 124, 140, 154, 162, 174, or tips thereof, after use.

36. The device 120, 130, 150, 160, 176 of claim 33 wherein the means are materials which are delivered into the hollow pathways to seal the microneedles 124, 140, 154, 162, 174.

## Patentansprüche

1. Vorrichtung (120, 130, 150, 160, 176) für einen Transport eines Materials oder von Energie über oder in eine verformbare, elastische, biologische Barriere, umfassend
a) mehrere hohle Mikronadeln (124, 140, 154, 162, 174), die jeweils ein Basisende und eine Spitze aufweisen, wobei mindestens ein hohler Pfad an oder zwischen dem Basisende und der Spitze angeordnet ist,
b) ein Substrat (122), an dem die Basisenden der Mikronadeln (124, 140, 154, 162, 174) befestigt oder integriert sind, und
c) mindestens ein Reservoir (126, 136), das bis zum Moment der Anwendung mit den Basisenden mindestens einer der Mikronadeln, entweder integriert oder trennbar, in Verbindung steht,
wobei das Volumen oder die Menge an Material, das zu transportieren ist, selektiv verändert werden kann, **dadurch gekennzeichnet, dass**
die Vorrichtung (120, 130, 150, 160, 176) Mittel (120, 132, 152, 164, 170) zum Verbessern des Durchdringens der Barriere durch die Mikronadeln (124, 140, 154, 162, 174) umfasst, indem sie die Elastizität der Barriere begrenzen.

2. Vorrichtung (120, 130, 150, 160, 176) nach Anspruch 1, wobei das Reservoir (126, 136) aus einem Material gebildet ist, das verformbar ist.

3. Vorrichtung (120, 130, 150, 160, 176) nach Anspruch 2, wobei das Reservoir (126, 136) elastisch ist.

4. Vorrichtung (120, 130, 150, 160, 176) nach Anspruch 1, wobei das Reservoir (126, 136) des Weiteren ein therapeutisches, prophylaktisches oder diagnostisches Agens umfasst.

5. Vorrichtung (120, 130, 150, 160, 176) nach Anspruch 4, wobei das Agens ausgewählt ist aus der Gruppe bestehend aus Peptiden, Proteinen, Kohlenhydraten, Nukleinsäuremolekülen, Lipiden, organischen Molekülen, biologisch aktiven, anorganischen Molekülen und Kombinationen davon.

6. Vorrichtung (120, 130, 150, 160, 176) nach Anspruch 1, des Weiteren umfassend einen Kolben (522), der beweglich an dem Substrat befestigt ist, wobei der Kolben (522) das Reservoir (126, 136) zusammenpressen kann.

7. Vorrichtung (120, 130, 150, 160, 176) nach Anspruch 6, des Weiteren umfassend eine Feder (534), die mit dem Kolben (522) in Eingriff steht.

8. Vorrichtung (120, 130, 150, 160, 176) nach Anspruch 1, wobei der Durchmesser der Mikronadeln (124, 140, 154, 162, 174) zwischen 10 µm und 200 µm beträgt.

9. Vorrichtung (120, 130, 150, 160, 176) nach Anspruch 1, wobei die Länge der Mikronadeln (124, 140, 154, 162, 174) zwischen 100 µm und 1 mm beträgt.

10. Vorrichtung (120, 130, 150, 160, 176) nach Anspruch 1, wobei die Mikronadeln (124, 140, 154, 162, 174) dazu ausgebildet sind, eine Einsetztiefe von weniger als 100 bis 150 µm bereitzustellen.

11. Vorrichtung (120, 130, 150, 160, 176) nach Anspruch 1, umfassend eine dreidimensionale Anordnung von Mikronadeln (124, 140, 154, 162, 174).

12. Vorrichtung (120, 130, 150, 160, 176) nach Anspruch 1, des Weiteren umfassend ein Klebematerial zur Befestigung der Vorrichtung (120, 130, 150, 160, 176) während der Abgabe.

13. Vorrichtung (120, 130, 150, 160, 176) nach Anspruch 1, wobei das Reservoir (126, 136) eine Spritze oder Pumpe ist, die mit dem Substrat verbunden ist.

14. Vorrichtung (120, 130, 150, 160, 176) nach Anspruch 1, umfassend ein Befestigungsmittel zum Befestigen der Vorrichtung an einer Spritze oder Pumpe.

15. Vorrichtung (120, 130, 150, 160, 176) nach Anspruch 13, wobei das Substrat dazu ausgebildet ist, entfernbar mit einer Standard- oder Luer-Lock-Spritze verbunden zu werden.

16. Vorrichtung (120, 130, 150, 160, 176) nach Anspruch 1, des Weiteren umfassend einen Dichtungsmechanismus (518), der zwischen dem Reservoir (126, 136) und dem Substrat (122) eingesetzt ist oder der an den Spitzen der Mikronadeln (124, 140, 154, 162, 174) befestigt ist.

17. Vorrichtung (120, 130, 150, 160, 176) nach Anspruch 16, wobei der Dichtungsmechanismus (518) eine brüchige Barriere ist, die zwischen dem Reservoir (126, 136) und dem Substrat (122) eingesetzt ist.

18. Vorrichtung (120, 130, 150, 160, 176) nach Anspruch 1, des Weiteren umfassend ein Mittel zum Bereitstellen einer Rückmeldung zur Anzeige, dass die Abgabe eingeleitet oder vollendet ist.

19. Vorrichtung (120, 130, 150, 160, 176) nach Anspruch 1, wobei mindestens die Mikronadeln (124, 140, 154, 162, 174) zur einmaligen Verwendung als wegwerfbares Reagens hergestellt sind.

20. Vorrichtung (120, 130, 150, 160, 176) nach Anspruch 1, wobei die Vorrichtung (120, 130, 150, 160, 176) des Weiteren Mittel zum Stoppen der Abgabe umfasst.

21. Vorrichtung (120, 130, 150, 160, 176) nach Anspruch 1, des Weiteren umfassend eine osmotische Pumpe zum Bewegen des Agens.

22. Vorrichtung (120, 130, 150, 160, 176) nach Anspruch 1, wobei die Mikronadel (124, 140, 154, 162, 174) in einem anderen Winkel als senkrecht zu dem Substrat (122) liegt.

23. Vorrichtung (120, 130, 150, 160, 176) nach Anspruch 1, wobei das Substrat (122) aus einem flexiblen Material gebildet ist.

24. Vorrichtung (120, 130, 150, 160, 176) nach Anspruch 1, umfassend mehrere Kammern oder Reservoirs (126, 136).

25. Vorrichtung (120, 130, 150, 160, 176) nach Anspruch 24, wobei die Kammern oder Reservoirs (126, 136) aneinander angeschlossen oder miteinander in Verbindung sein können.

26. Vorrichtung (120, 130, 150, 160, 176) nach Anspruch 24, wobei ein oder mehrere Reservoirs (126, 136) eine feste Formulierung enthalten und ein oder mehrere Reservoirs (126, 136) einen flüssigen Träger für die feste Formulierung enthalten.

27. Vorrichtung (120, 130, 150, 160, 176) nach Anspruch 1, in einer Verpackung, die die Vorrichtung (120, 130, 150, 160, 176) vor einer Beschädigung und/oder Verunreinigung schützt.

28. Vorrichtung (120, 130, 150, 160, 176) nach Anspruch 1, des Weiteren umfassend eine entfernbare Schutzfolie, die die Mikronadelspitzen bedeckt und die zum Schützen und/oder Abdichten des Zugangs zu oder von den Mikronadeln (124, 140, 154, 162, 174) verwendet werden kann.

29. Vorrichtung (120, 130, 150, 160, 176) nach Anspruch 1, wobei die Vorrichtung (120, 130, 150, 160, 176) Ratenkontrollmittel umfasst, die zum Regulieren der Rate oder des Ausmaßes verwendet werden können, mit der beziehungsweise in dem Materialien durch die Mikronadeln (124, 140, 154, 162, 174) strömen.

30. Vorrichtung (120, 130, 150, 160, 176) nach Anspruch 29, wobei das Ratenkontrollmittel eine Membran an einem Ende der Mikronadeln (124, 140, 154, 162, 174) ist, durch die ein Agens oder Fluid hindurchgehen muss.

31. Vorrichtung (120, 130, 150, 160, 176) nach Anspruch 29, wobei das Ratenkontrollmittel ein Material ist, das in dem hohlen Pfad der Mikronadeln (124, 140, 154, 162, 174) angeordnet ist.

32. Vorrichtung (120, 130, 150, 160, 176) nach Anspruch 1, umfassend mehrere Mikronadelanordnungen in Kombination mit Mitteln für einen gleichzeitigen Zugang zu einer oder mehreren Anordnungen.

33. Vorrichtung (120, 130, 150, 160, 176) nach Anspruch 1, umfassend Mittel zum Verhindern eines unerwünschten Kontakts mit den oder einer Verwendung der Mikronadeln (124, 140, 154, 162, 174).

34. Vorrichtung (120, 130, 150, 160, 176) nach Anspruch 33, wobei das Mittel eine Verpackung ist, die die Mikronadeln (124, 140, 154, 162, 174) bedeckt.

35. Vorrichtung (120, 130, 150, 160, 176) nach Anspruch 33, wobei das Mittel eine Verpackung ist, die die Mikronadeln (124, 140, 154, 162, 174) oder deren Spitzen nach dem Gebrauch abbricht.

36. Vorrichtung (120, 130, 150, 160, 176) nach Anspruch 33, wobei das Mittel Materialien sind, die in den hohlen Pfad zum Abdichten der Mikronadeln (124, 140, 154, 162, 174) abgegeben werden.

## Revendications

1. Dispositif (120, 130, 150, 160, 176) pour transporter un matériau ou de l'énergie à travers ou dans une barrière biologique déformable et élastique, comprenant :
a) une pluralité de micro-aiguilles creuses (124, 140, 154, 162, 174) ayant chacune une extrémité de base et une pointe, avec au moins une voie d'accès creuse disposée au niveau de ou entre l'extrémité de base et la pointe,
b) un substrat (122) auquel les extrémités de base des micro-aiguilles (124, 140, 154, 162, 174) sont attachées ou intégrées, et
c) au moins un réservoir (126, 136) qui est en connexion avec les extrémités de base d'au moins une des micro-aiguilles, en étant soit intégré soit séparé jusqu'au moment de l'utilisation,
dans lequel le volume ou la quantité de matériau devant être transporté peut être sélectivement modifié(e), **caractérisé en ce que** le dispositif (120, 130, 150, 160, 176) comprend des moyens (128, 132, 152, 164, 170) pour améliorer la pénétration de la barrière par les micro-aiguilles (124, 140, 154, 162, 174) en limitant l'élasticité de la barrière.

2. Dispositif (120, 130, 150, 160, 176) selon la revendication 1, dans lequel le réservoir (126, 136) est formé d'un matériau qui est déformable.

3. Dispositif (120, 130, 150, 160, 176) selon la revendication 2, dans lequel le réservoir (126, 136) est élastique.

4. Dispositif (120, 130, 150, 160, 176) selon la revendication 1, dans lequel le réservoir (126, 136) comprend en outre un agent thérapeutique, prophylactique ou diagnostique.

5. Dispositif (120, 130, 150, 160, 176) selon la revendication 4, dans lequel l'agent est sélectionné dans le groupe consistant en des peptides, des protéines, des glucides, des molécules d'acide nucléique, des lipides, des molécules organiques, des molécules inorganiques biologiquement actives, et des combinaisons de ceux-ci.

6. Dispositif (120, 130, 150, 160, 176) selon la revendication 1, comprenant en outre un piston (522) fixé de façon amovible au substrat, où le piston (522) peut comprimer le réservoir (126, 136).

7. Dispositif (120, 130, 150, 160, 176) selon la revendication 6, comprenant en outre un ressort (534) engagé avec le piston (522).

8. Dispositif (120, 130, 150, 160, 176) selon la revendication 1, dans lequel le diamètre des micro-aiguilles (124, 140, 154, 162, 174) est compris entre 10 µm et 200 µm.

9. Dispositif (120, 130, 150, 160, 176) selon la revendication 1, dans lequel la longueur des micro-aiguilles (124, 140, 154, 162, 174) est comprise entre 100 µm et 1 mm.

10. Dispositif (120, 130, 150, 160, 176) selon la revendication 1, dans lequel les micro-aiguilles (124, 140, 154, 162, 174) sont adaptées pour fournir une profondeur d'insertion de moins de 100 à 150 µm.

11. Dispositif (120, 130, 150, 160, 176) selon la revendication 1, comprenant un réseau de micro-aiguilles (124, 140, 154, 162, 174) tridimensionnel.

12. Dispositif (120, 130, 150, 160, 176) selon la revendication 1, comprenant en outre un matériau adhésif pour fixer le dispositif (120, 130,150, 160, 176) pendant la délivrance.

13. Dispositif (120, 130, 150, 160, 176) selon la revendication 1, dans lequel le réservoir (126, 136) est une seringue ou une pompe connectée au substrat.

14. Dispositif (120, 130, 150, 160, 176) selon la revendication 1, comprenant un moyen d'attachement pour fixer le dispositif à une seringue ou à une pompe.

15. Dispositif (120, 130, 150, 160, 176) selon la revendication 13, dans lequel le substrat est adapté pour se connecter de façon amovible à une seringue standard ou Luer-lock.

16. Dispositif (120, 130, 150, 160, 176) selon la revendication 1, comprenant en outre un mécanisme d'étanchéité (518) qui est interposé entre le réservoir (126, 136) et le substrat (122) ou qui est fixé aux pointes des micro-aiguilles (124, 140, 154, 162, 174).

17. Dispositif (120, 130, 150, 160, 176) selon la revendication 16, dans lequel le mécanisme d'étanchéité (518) est une barrière fracturable interposée entre le réservoir (126, 136) et le substrat (122).

18. Dispositif (120, 130, 150, 160, 176) selon la revendication 1, comprenant en outre un moyen pour obtenir des informations indiquant que la délivrance est initiée ou terminée.

19. Dispositif (120, 130, 150, 160, 176) selon la revendication 1, dans lequel au moins les micro-aiguilles (124, 140, 154, 162, 174) sont fabriquées pour un usage unique en tant que réactif jetable.

20. Dispositif (120, 130, 150, 160, 176) selon la revendication 1, où le dispositif (120, 130, 150, 160, 176) comprend en outre un moyen pour arrêter la délivrance.

21. Dispositif (120, 130, 150, 160, 176) selon la revendication 1, comprenant en outre une pompe osmotique pour déplacer un agent.

22. Dispositif (120, 130, 150, 160, 176) selon la revendication 1, dans lequel la micro-aiguille (124, 140, 154, 162, 174) est orientée selon un angle autre qu'un angle perpendiculaire par rapport au substrat (122).

23. Dispositif (120, 130, 150, 160, 176) selon la revendication 1, dans lequel le substrat (122) est formé d'un matériau souple.

24. Dispositif (120, 130, 150, 160, 176) selon la revendication 1, comprenant de multiples compartiments ou réservoirs (126, 136).

25. Dispositif (120, 130, 150, 160, 176) selon la revendication 24, dans lequel les compartiments ou réservoirs (126, 136) peuvent être connectés ou en communication les uns avec les autres.

26. Dispositif (120, 130, 150, 160, 176) selon la revendication 24, dans lequel au moins un réservoir (126, 136) contient une formulation solide et au moins un réservoir (126, 136) contient un véhicule liquide pour la formulation solide.

27. Dispositif (120, 130, 150, 160, 176) selon la revendication 1, qui est dans un conditionnement qui protège le dispositif (120, 130, 150, 160, 176) des dommages et/ou d'une contamination.

28. Dispositif (120, 130, 150, 160, 176) selon la revendication 1, comprenant en outre un revêtement amovible qui recouvre les pointes des micro-aiguilles et qui peut être utilisé pour protéger et/ou empêcher, de façon hermétique, tout accès aux ou à partir des micro-aiguilles (124, 140, 154, 162, 174).

29. Dispositif (120, 130, 150, 160, 176) selon la revendication 1, dans lequel le dispositif (120, 130, 150, 160, 176) comprend un moyen de contrôle du débit, qui peut être utilisé pour réguler le débit ou la mesure dans laquelle des matériaux circulent dans les micro-aiguilles (124, 140, 154, 162, 174).

30. Dispositif (120, 130, 150, 160, 176) selon la revendication 29, dans lequel le moyen de contrôle du débit est une membrane située à une extrémité des micro-aiguilles (124, 140, 154, 162, 174), à travers laquelle doit passer un agent ou un liquide.

31. Dispositif (120, 130, 150, 160, 176) selon la revendication 29, dans lequel le moyen de contrôle du débit est un matériau positionné au sein de la voie d'accès creuse des micro-aiguilles (124, 140, 154, 162, 174).

32. Dispositif (120, 130, 150, 160, 176) selon la revendication 1, comprenant de multiples réseaux de micro-aiguilles en combinaison avec un moyen pour accéder à un ou plusieurs réseaux en même temps.

33. Dispositif (120, 130, 150, 160, 176) selon la revendication 1, comprenant un moyen pour prévenir un contact indésirable avec, ou une utilisation des micro-aiguilles (124, 140, 154, 162, 174).

34. Dispositif (120, 130, 150, 160, 176) selon la revendication 33, dans lequel le moyen est un conditionnement qui recouvre les micro-aiguilles (124, 140, 154, 162, 174).

35. Dispositif (120, 130, 150, 160, 176) selon la revendication 33, dans lequel le moyen est un conditionnement qui permet de détacher les micro-aiguilles (124, 140, 154, 162, 174), ou leurs pointes, après utilisation.

36. Dispositif (120, 130, 150, 160, 176) selon la revendication 33, dans lequel le moyen est des matériaux qui sont délivrés dans les voies d'accès creuses afin de sceller les micro-aiguilles (124, 140, 154, 162, 174).
